# EUROPEAN PATENT APPLICATION

(11) **EP 0 857 456 A1**
(43) Date of publication of application: **12.08.1998**
(21) Application number: 97308986.5
(22) Date of filing: 10.11.1997
(51) Int. Cl.: A61B 5/00, H04N 7/18, A61J 7/00

(54) **Interactive medication delivery system with visual patient monitoring**

(30) Priority: 15.11.1996 US 751005
(71) Applicant: HealthTech Services Corporation, Northbrook, IL 60062 (US)
(72) Inventor: Kaufman, Stephen B., Highland Park, Illinois 60035 (US); Stanton, Michael K., Hoffman Estates, Illinois 50195 (US)
(74) Representative: MacGregor, Gordon

(57) **Abstract**

A medication dispensing device has a housing (12) with a location (40,42) for holding at least one dose of a medication for the user. The device prompts the user to take the medication held by it. A video camera (306) on the housing records visual images of the user in response to a prescribed command. The device assists the user in following a prescribed medication regime. The device also visually monitors and records the user's demeanor, physical appearance, and general well-being for analysis at a remote patient-care monitoring facility.

## Description

### Related Applications

This is a continuation-in-part of pending U.S. Application Serial No. 08/563,129, filed November 27, 1995, which is a continuation of now abandoned U.S. Application Serial No. 08/355,431 filed December 13, 1994, which is a continuation of now abandoned U.S. Application Serial No. 08/126,178 filed September 23, 1993.

### Field of the Invention

The invention generally relates to systems for dispensing medications. In a more particular sense, the invention concerns systems which oversee and coordinate the administration of complex medication regimens at home, outside the hospital or pharmacy, and without the day to day supervision of medical personnel. In this more particular sense, the invention also concerns automated home care patient health monitoring systems.

### Background of the Invention

Due to advances in medicine and medical treatments in general, people are living longer. As a result, the number and percentage of older people (referred to as "the elderly") are growing in the United States and elsewhere.

However, despite medical advances, many elderly still face chronic and debilitating health problems. Arthritis, hypertension, and heart conditions are but a few examples of the problems associated with longevity.

Treatment of these health problems often requires close compliance with relatively complex medication regimes. It is not unusual for a person having one of the above health problems to be taking four or more different prescription drugs at one time. These drugs often differ significantly in dosages, both as to time and amount, as well as in their intended physiological effects. These drugs also often differ in the severity of potentially adverse reactions due to mismedication.

Close and careful compliance with these complex medication regimes is a difficult task in itself. The difficulty is greatly enhanced, considering that the elderly must discipline themselves to follow these regimes at home, without the day-to-day support and supervision of trained hospital and pharmacy personnel, and often without the day-to-day support and supervision of their immediate families or other care givers. Furthermore, a loss in short term memory can be naturally attributed to the aging process and to the medication themselves, resulting in forgetfulness and further confusion in scheduling compliance with complicated medication regimes.

The elderly are therefore increasingly at risk of hospitalization or death from mismedication.

An interactive patient assistance devices, ideally suited to the needs of home care patients -- young and old alike -- is described in Kaufman et al. U.S. Patents 4,933,873; 5,124,484; 5,084,828; and 5,126,957. These devices include a self-contained medication delivery mechanism and self-contained physical testing apparatus. They normally retain the medication and the testing apparatus away from access by the patient. Both medication and the testing apparatus are made available to the patient, either in response to a prescribed schedule or in response to a verbal command made by the patient. In some embodiments, the devices differentiate between medications that are to be administered strictly according to a schedule prescribed by a physician and those that can be administered upon patient demand.

The present invention enhances and expands the flexible, interactive system described in the above-identified Kaufman et al. patents.

The invention is directed to improving the overall well-being and lifestyle of home care patients who are on complicated medication regimes. The invention addresses the problems of compliance with a complicated regime of differing medications and solves these problems by addressing the needs for self-sufficiency and personal control without sacrificing the overall therapeutic objectives of the prescribed medical treatment.

### Summary of the Invention

One aspect of the invention provides an apparatus for dispensing medication. The device has a housing with a location for holding at least one dose of a medication for the user. The device prompts the user to take the medication held by it. A video camera on the housing records visual images of the user in response to a prescribed command.

The device is therefore able, not only to assist the user in following a prescribed medication regime, but also to visually monitor and record the user's demeanor, physical appearance, and general well-being.

In a preferred embodiment, the device stores in memory both the taking of medication by the user over a selected time period, as well as at least some of the recorded visual images taken over the same selected time period.

In a preferred embodiment, the device transmits at least a portion of the recorded images to an external monitoring facility. In this arrangement, the device also transmits to the external monitoring facility the record of the taking of medication by the user.

The device thereby creates both a quantitative and visual record while monitoring the user's compliance with a prescribed medication regime.

Another aspect of the invention provides an apparatus for dispensing a physiologic testing device. The apparatus includes a housing that holds a testing device for measuring a prescribed physiologic parameter of the user. The apparatus also includes a video camera on the housing that operates in response to a prescribed command for recording visual images of the user of the testing device.

In a preferred embodiment, the apparatus stores in memory the physiologic parameters measured by the testing device over a selected time period. The apparatus also stores in memory at least some of the recorded visual images taken over the same selected time period.

Therefore, the apparatus not only assists the user periodically testing one or more physiologic parameters, but also provides a visual record of the user's demeanor, physical appearance, and general well-being.

In a preferred embodiment, the apparatus transmits at least some the recorded images to an external monitoring facility. The apparatus also transmits to the external monitoring facility the physiologic parameters measured by the testing device.

The device thereby creates both a quantitative and visual record of the physiologic parameters and appearance of the user.

In a preferred embodiment, both aspects of the invention augment the camera's function by providing a motor actuated swivel mechanism that holds the camera and rotates the camera relative to the housing in response to a prescribed command.

In a preferred embodiment, the swivel mechanism is activated in response to sound to rotate the camera, as well as in response to a command sent by an external monitoring facility.

In a preferred embodiment, the apparatus includes a control mechanism that issues a command to actuate the camera is response to prescribed criteria. For example, the control mechanism can actuate the camera based upon indications of an abnormal heath condition for the user based upon medication usage or measurements of physiologic parameters.

Both aspects of the invention also augment the camera's function in a preferred embodiment by providing a zoom lens for altering the focal plane of the visual image in response to a prescribed command, either local or remote.

In one embodiment, the patient monitoring camera is contained in a module mounted on the housing of the device. Preferably, the module is detachably coupled to one end of an articulated arm. The module is received in a recess formed in the housing and is movable between a retracted position and an extended position. The articulated arm enables the camera to be aimed where desired.

In one embodiment, the module is pivotable at the end of the articulated arm. The module can be detached from the arm to further facilitate visual monitoring of the patient. Preferably, a pair of adjustable legs on the camera module enable the camera module to be supported on, and positioned relative to, a supporting surface.

In one embodiment, the camera is equipped with a light ring for illuminating the subject matter to be imaged with the camera. Preferably, the light ring surrounds the lens of the camera and provides continuous illumination.

In one embodiment, the camera is used as part of a prescribed treatment protocol administered to the patient. In particular, the camera is used to record patient compliance with various directives. In addition, the camera is used in the visual inspection of certain physiologic conditions such as wound condition and the condition of intravenous sites.

The various aspects of the invention integrate qualitative and visual monitoring of a person's health and well being. The invention provides a "smart," interactive system that gives personalized heath care assistance without requiring a constant human presence.

Other features and advantages of the invention will become apparent upon reviewing the following detailed description, drawings, and appended claims.

### Brief Description of the Drawings

Fig. 1 is a front perspective view of a patient monitoring and assistance device having an enclosed system for delivering physiologic testing devices to the patient, as well as an enclosed medication storage and dispensing system that embodies the features of the invention for storing and dispensing medication in individual caplets, each of which systems is shown in its closed position;
Fig. 2 is a front perspective view of the device shown in Fig. 1, with the testing device delivery system and medication delivery system each shown in its open position;
Fig. 3 is an enlarged perspective view, with portions broken away, of the interior of the device shown in Fig. 2, showing the enclosed medication storage and dispensing system;
Fig. 4 is a schematic block diagram of the system that controls the operation of the patient assist device shown in Fig. 1;
Fig. 5 is a schematic and partially diagrammatic block diagram of the elements of the system shown in Fig. 4 that control the operation of the medication delivery system that incorporates the invention;
Figs. 6 to 9 are schematic and partially diagrammatic flow charts of differing embodiments of the system for controlling the operation of the medication delivery system that incorporate aspects of the invention;
Fig. 10 is an enlarged side view, partially broken away, of the medication delivery system shown in Fig. 3, tipped outwardly from the rear of the associated patient assist device for replenishment of medication;
Fig. 11 is an enlarged side view, partially broken away, of the medication delivery system shown in Fig. 3 in its operative position within the associated patient assist device;
Figs. 12 to 14 are enlarged side views of the medication delivery system taken generally along line 12-12 in Fig. 3, showing the sequence of operation in dispensing medication in caplet form;
Fig. 15 is a front perspective view of the medication delivery system shown in Fig. 3, opened to permit replenishment of the storage compartments in modular form;
Fig. 16 is a schematic and partially diagrammatic view of a medication inventory and management system that embodies the features of the invention;
Fig. 17 is a schematic and partially diagrammatic view of medication files contained in the inventory and management system shown in Fig. 14;
Fig. 18 is a schematic and partially diagrammatic flow chart of a medication administration follow up sequence that forms a part of the inventory and management system shown in Fig. 14;
Fig. 19 is a front perspective view of an alternative embodiment of a patient monitoring and assistance device that includes a visual patient monitoring system;
Fig. 20 is an enlarged perspective view, partially broken away to show interior portions, of the visual patient monitor system shown in Fig. 19;
Fig. 21 is an enlarged perspective view of the visual patient monitoring system shown in Fig. 19 as it pivots to scan the room where it is located;
Fig. 22 is an close up view of the use of the diagnostic video camera that forms a part of the visual patient monitoring system shown in Fig. 19; and
Fig. 23 is a diagrammatic view of the link between the visual patient monitoring system shown in Fig. 19 and a central monitoring facility.
Fig. 24 is a fragmentary perspective view of still another alternate embodiment of a patient monitoring and assistance device that includes a visual patient monitoring system showing a patient monitoring camera in an extended, use position.
Fig. 25 is a fragmentary perspective view similar to Fig. 24 showing the patient monitoring camera in a retracted, storage position.
Fig. 26 is a fragmentary perspective view similar to Figs. 24 and 25 showing the patient monitoring camera detached from the main patient monitoring and assistance device unit.
Fig. 27 is a fragmentary side-elevation view, partially in section, showing an articulated arm assembly used to mount the patient monitoring camera to the main patient monitoring and assistance device unit.
Fig. 28 is a cross-sectional view of the articulated arm assembly shown in Fig. 28 taken along line 28-28 thereof.
Fig. 29 is a facsimile of a screen display generated in one embodiment of the patient monitoring and assistance device guiding the patient in use of the patient monitoring camera.
Fig. 30 is a diagrammatic view of a patient assistance device that incorporates an image acquiring element to aid in assuring use and health compliance criteria are met.
Fig. 31 is a diagrammatic view of the use compliance component of the device shown in Fig. 30.
Fig. 32 is a diagrammatic view of the health compliance component of the device shown in Fig. 30.

### Description of the Preferred Embodiments

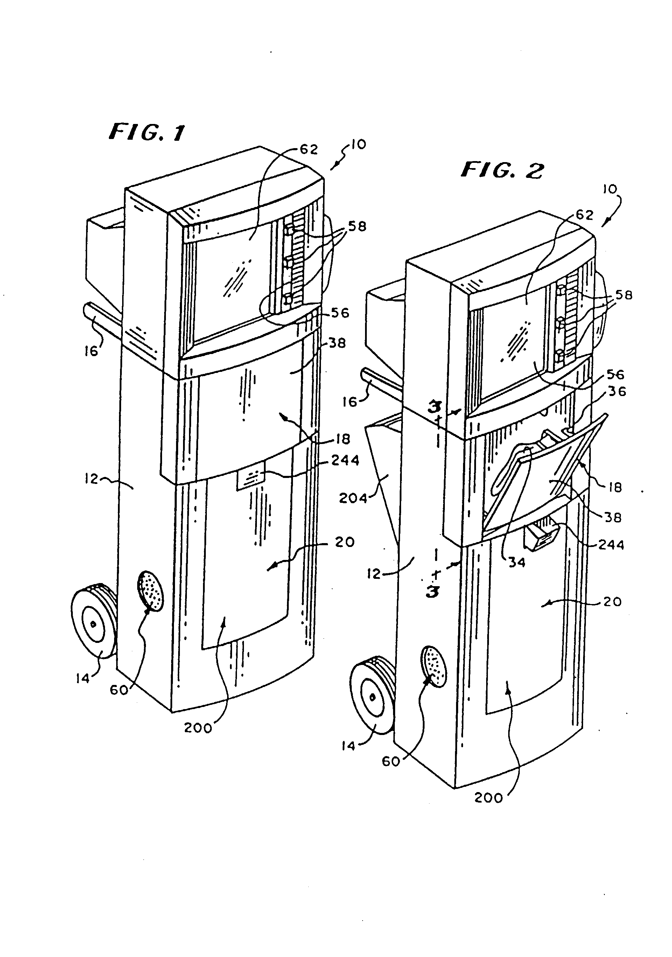

One preferred embodiment of an interactive monitoring and assistance device 10 is shown in Figs. 1 and 2. As will soon be described in greater detail, the device 10 performs as a self-contained, microprocessor-based caregiver who, in a friendly and supportive manner, monitors, manages and assists a patient in performing everyday health maintenance tasks. The device 10 monitors the patient's vital signs. The device 10 also stores and administers medication. The device 10 preferably is linked to a central facility that provides round-the-clock supervision and response as required.

The device 10 includes a housing or cabinet 12 that, in a preferred design, stands about four feet tall. Preferably, the housing 12 is portable. For this purpose, the device 10 includes wheels 14 and a handle 16 for the patient, or another user, to guide the movement.

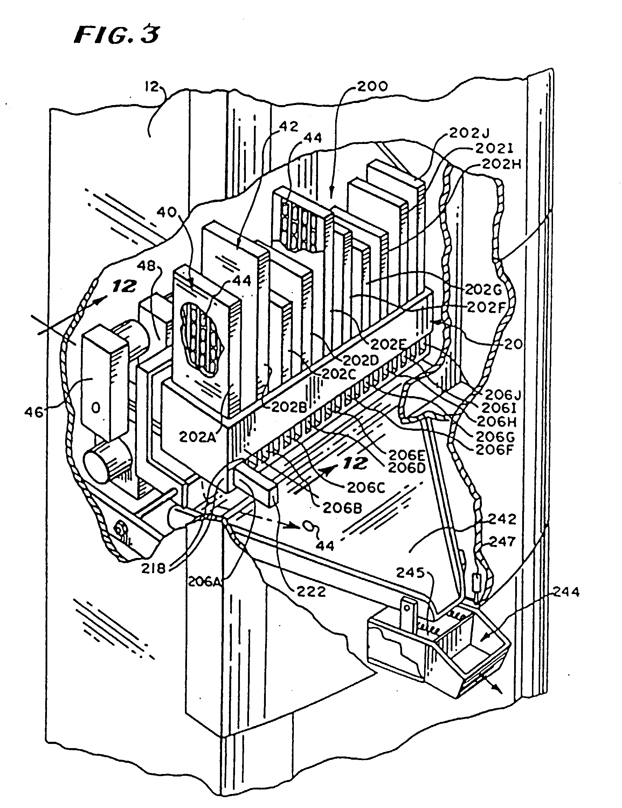

As shown in Figs. 1 and 2, the device 10 houses a system 18 for storing and delivering one or more devices for testing the vital signals of a patient. The device 10 also houses a system 20 for storing and administering medication (see Fig. 3 also).

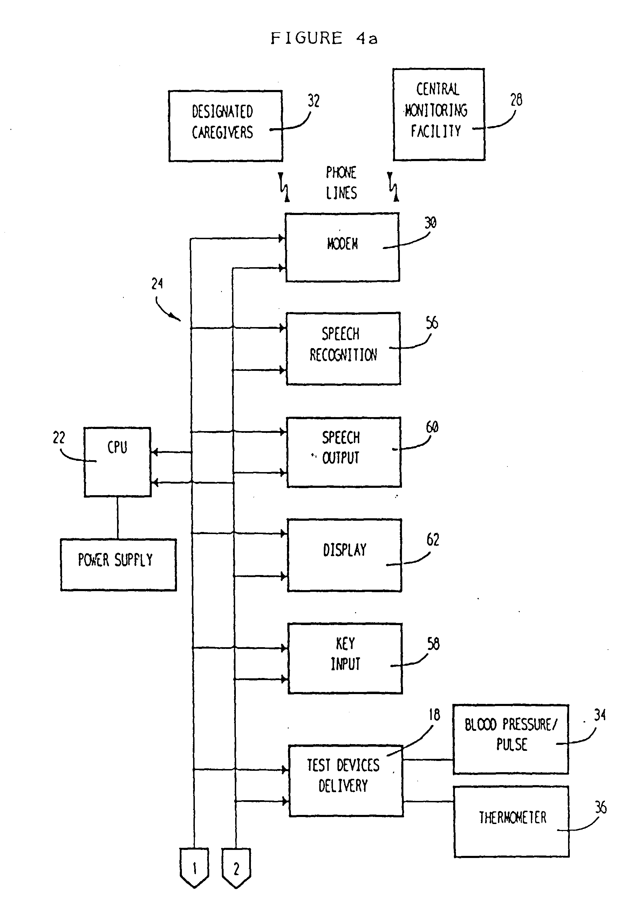

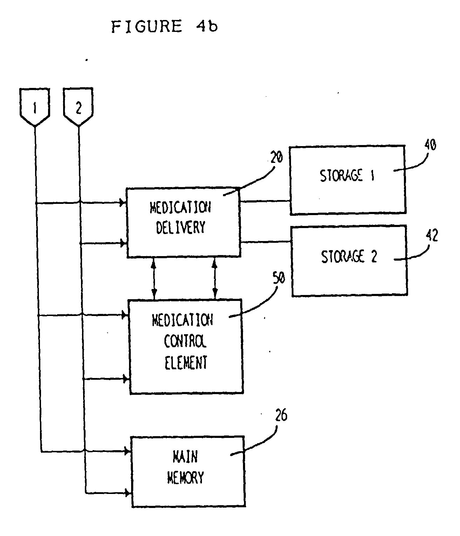

In the illustrated and preferred embodiment shown in Fig. 4, the device 10 houses a main microprocessor-based CPU 22 that coordinates and controls its operation. While various arrangements are possible, the CPU 22 preferably comprises a conventional CPU board, for example, an IBM PC compatible CPU board. Various input/output (I/O) devices communicate with the main CPU 22 through conventional data and address busses 24. The I/O devices will be described in greater detail later. A mass storage device for storing digital information also communicates with the main CPU 22 through the busses 24.

In use, as shown in Fig. 4, the device 10 is preferably linked with a central monitoring facility 28 by a modem 30 that communicates with the main CPU 22 through the busses 24. Health care professionals are present on a 24 hour basis at the central facility 28 to monitor the health of the patient based upon information collected and transmitted to them by the device 10. The device 10 is also preferably linked via the modem 30 with selected individuals 32 -- typically close friends, family members, or other designated caregivers -- who are automatically notified by the device 10 when certain health conditions exist or upon request by the patient or central facility 28.

As shown in Fig. 2, the system 18 for monitoring the patient's vital signs includes one or more physiologic testing devices, which, for the purpose of illustration, are a pressure cuff 34 for measuring blood pressure and pulse rate, and a thermometer 36 for measuring body temperature. Of course, other testing devices could be provided, depending upon the health condition of the patient and mode of treatment. For example, other testing devices can include a pulse oximeter, an EKG monitor, or a digital stethoscope.

As shown in Fig. 4, the testing devices 34 and 36 communicate with the main CPU 22 through the busses 24. The measurements taken are stored in the data storage device 26. These measurements are also periodically transmitted to the central monitoring facility 28 by the modem 30. The central facility 28 also preferably records received information in its own mass storage device for record keeping, retrieval and analysis.

Preferably, the testing devices 34 and 36 are housed in a movable compartment or drawer 38 within the housing 12. The drawer 38 is normally closed (as shown in Fig. 1), thereby retaining the testing devices 34 and 36 within the housing 12 away from access by the patient. The drawer 38 will open in response to an appropriate command signal received and interpreted by the main CPU 22. The opened position for the drawer 38 is shown in Fig. 2. The testing devices 34 and 36 are thereby made available for use by the patient. This particular operation will be described in greater detail later.

The medication delivery system 20, housed within the device 10 (see Fig. 3), embodies the features of the invention. The system 20 is capable of storing and administering different types of medications having different administration criteria. The criteria can differ in terms of prescribed dosage amount, prescribed frequency of administration, degree of accessibility to the patient, or various combinations of the above.

The medication delivery system 20 includes at least two discrete storage compartments or cassettes (generally designated 40 and 42 in Fig. 3) within the housing 12. Each storage compartment 40 and 42 is isolated from the other storage compartment, and each is capable of separately storing at least one dose of a medication 44 within the housing 12 away from access by the user.

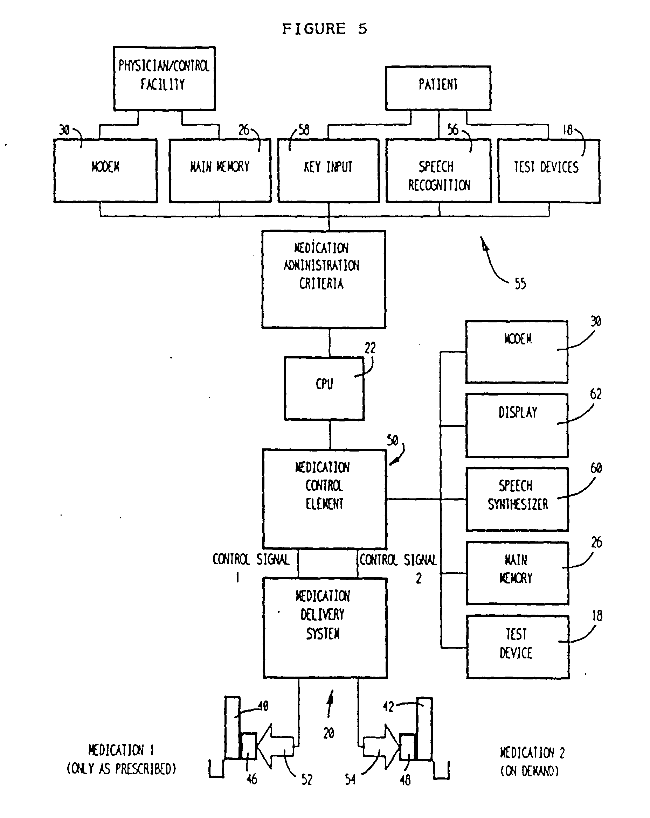

The medication delivery system 20 further includes independent delivery means or mechanisms associated with each storage compartment. In the illustrated arrangement (see Figs. 3 and 5), a first delivery mechanism 46 is associated with the first storage compartment 40 for selectively delivering a medication dose from there to the patient. A second delivery mechanism 48 is likewise associated with the second storage compartment 42 for selectively delivering a medication dose from there to patient. The number of individual delivery systems corresponds with the number of individual medication storage compartments. The number of storage compartments can, of course, vary. Only two storage compartments and their associated delivery systems will be discussed.

The first and second delivery mechanisms 46 and 48 operate independently and in response to different administration criteria. For this purpose (in particular, see Fig. 5), the medication delivery system 20 includes a control means or element 50 associated with the first and second delivery mechanisms 46 and 48. In the illustrated and preferred embodiment, the control element 50 communicates with the main CPU 22 (see Fig. 4, too), either in the form of programmable random access memory (RAM) device or as preprogrammed read only memory (ROM) device.

According to its programming, the control element 50 is capable of receiving and differentiating between at least two different prescribed inputs. Upon the receipt and interpretation a first prescribed input or combination of inputs, the control element 50 will generate a control signal 52 that actuates the first delivery mechanism 46. Upon receipt of the second prescribed input or combination of inputs different from the first input, the control element 50 will generate a control signal 54 that actuates the second delivery mechanism 48. The control element 50 will not actuate the first delivery mechanism 46 in response to the second prescribed input.

The first and second control signals 52 and 54 are generated in response to different prescribed input criteria, and medications stored in the two storage compartments 40 and 42 can thereby be selectively administered differently.

As best shown in Fig. 5, the input criteria that generate the first and second control signals are derived from both external and internal devices associated with the medication delivery system 20, and in this way also from both the physician (or healthcare professional) and the individual patient.

More particularly, the system 20 includes in internal memory 26 one or more prescribed schedules for administering medication. Here, the attending physicians records the prescribed medication regime for the patient.

The system 20 also includes various external input devices for receiving and interpreting prescribed commands either from the patient or from the central monitoring facility 28. These external input devices communicate with the control element 50 through the main CPU 22 (see Fig. 4). The received commands can include one or more specified commands for administering medication "upon demand".

In the illustrated and preferred embodiment shown in Figs. 4 and 5, the external input devices include a speech recognition system 56 for receiving and interpreting preselected verbal commands made by the patient (for example, a Texas Instruments Recognition and Speech Unit Model TI-2245186-001). The external input devices also include the modem 30 for receiving and interpreting preselected commands from the central facility 28.

In addition, the external input devices preferably include one or more input keys 58 located at a user-convenient place on the housing 12, allowing the patient to manually enter the prescribed commands, if desired. In the illustrated and preferred embodiment shown in Figs. 1 and 2, only a select few input keys 58 for entering block (or macro-) commands are provided in the interest of simplifying the patient's interface with the device 10. However, it should be appreciated that a full keyboard could also be included, depending upon the degree of sophistication and desires of the patient.

In the illustrated and preferred embodiment shown in Figs. 4 and 5, the system also includes an external output device associated with the main CPU 22 for delivering messages or otherwise communicating with the patient. Preferably, the external output device includes a speech synthesizing system 60 for synthesizing audible messages to the user. In addition, the external output device also preferably includes a video monitor 62 on which the audible messages appear in written form (see Figs. 1 and 2 also). In this arrangement, the video monitor 62 can also display in written form the preselected medication administration commands. In this arrangement, the video monitor 62 serves to visually back up and confirm the verbal messages and commands being exchanged by the patient and the device 10, thereby minimizing the chance of misunderstandings or failures to communicate.

Due to these various input and output devices, the medication delivery system 20 as just described affirmatively interacts with the patient, relying upon both spoken and written forms of communication with the patient.

### System for Storing and Administering Scheduled Medication and On Demand Medication

In accordance with one aspect of the invention, the control element 50 as above described can store and selectively administer one category of medication that should be administered only according to a prescribed schedule and another category of medication that can be administered upon demand by the patient.

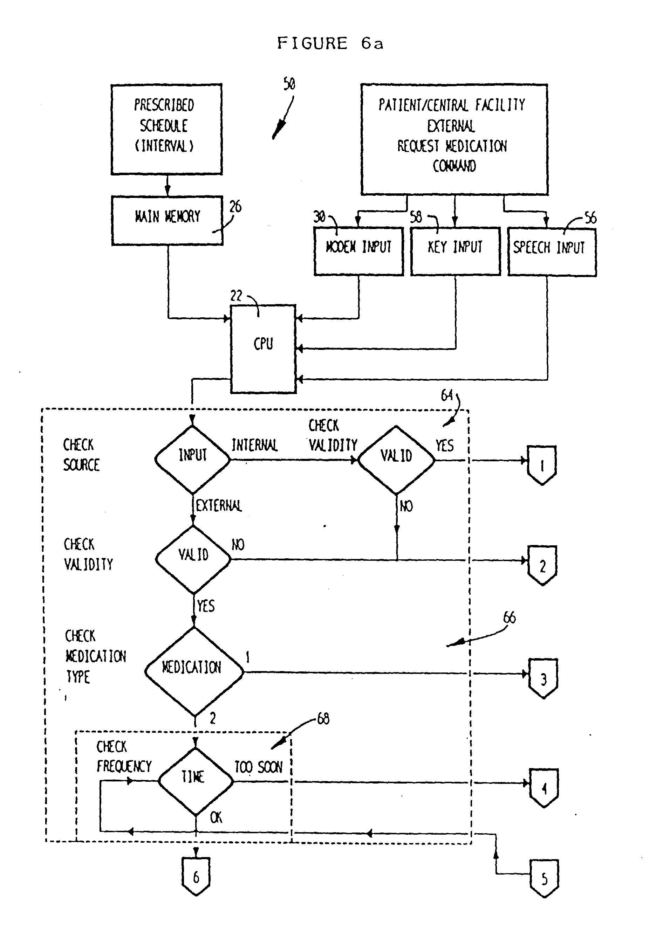

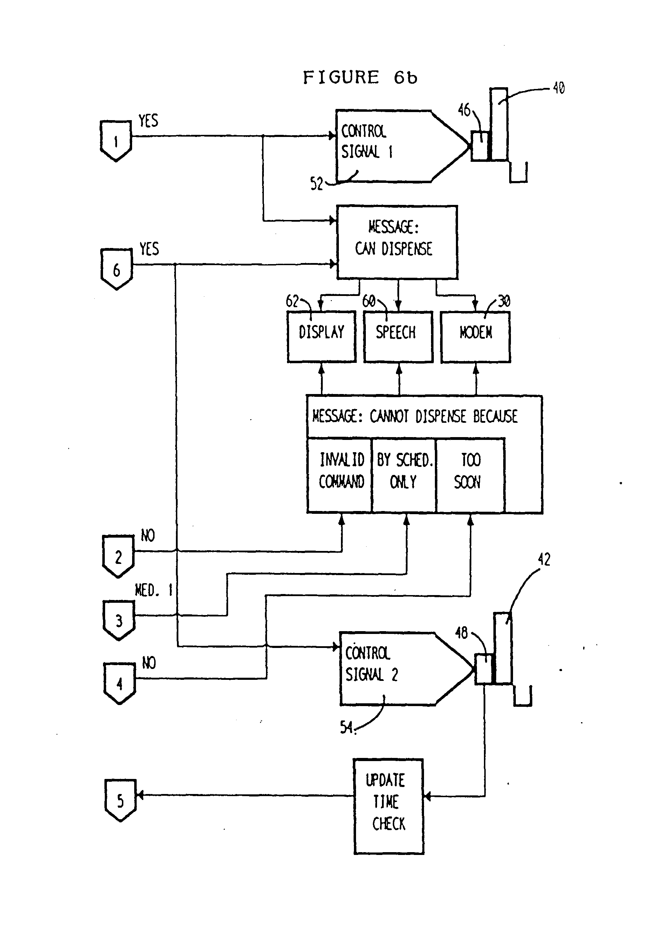

The control element 50 associated with this arrangement is shown diagrammatically in Fig. 6. The prescribed medication schedule is retained in the internal memory 26. The control element 50 includes a first operative sequence 64 that, upon receiving a valid administer medication command from an internal source (that is, internal to the system 20), will generate the first control signal 52. The appropriate administer medication command is internally issued periodically by the CPU 22, based upon a comparison of real time with the prescribed medication schedule stored in the internal memory 26.

Upon generation of the first control signal 52, medication retained in the first storage compartment 40, and only the first storage compartment 40, will be released to the patient.

Preferably, the first operative sequence 64 also generates a "Can Administer" message, using one or more of the output devices (the speech synthesizer 60 and/or the display 62), advising the patient that it is time to take the prescribed medication.

The control element 50 also includes a second operative sequence 66 that, in association with the external input devices (modem 30/key input 56/speech recognition 58), receives and interprets one or more medication delivery commands received from an external source, such as the patient or the central facility 28. As shown in Fig. 6, the second operative sequence 66 conducts a validity check upon the command. The second operative sequence 66 also checks to determine what type or category of medication is being requested.

Upon receipt of the proper (valid) command or commands requesting the proper type of medication, the second operative sequence 66 generates the second control signal 54. The medication retained in the second storage compartment 42, but not the first storage compartment 40, is thereby released to the patient. As before, the second operative sequence 66 also preferably communicates an appropriate "Can Administer" message to the patient through one or more of the output devices 60/62. If the medication request originates from the patient, an advisory message may also be sent to the central facility 28 via the modem 30 at the time an "on demand" request is received and implemented.

If an invalid command is received, or if the patient requests a medication that can only be administered according to an internal command from the internal memory, an appropriate "Cannot Dispense" message is display and/or spoken using the output devices 60/62.

The first delivery mechanism 46 is thereby actuated in response to an internally generated command signal, but not in response to an externally generated command signal. The first category of medication can thus be safely retained within the first storage compartment 40 away from patient access, except as controlled by the control element 50 (via the first control signal 52). Strict compliance with the prescribed medication schedule is assured.

The second delivery mechanism 48 is actuated in response to the second control signal 54 based upon externally received commands. The second category of "on demand" medication can thus be safely retained in the second storage compartment 42 for administration externally controlled by the patient or the central facility 28 by issuing a proper external command.

In the illustrated and preferred embodiment shown in Fig. 6, the control element 50 also includes a third operative sequence 68 that maintains a real time record of "on demand" administrations of medication and the elapsed time period therebetween. The third operative sequence 68 includes timing means 70 for comparing the elapsed time between one actuation and the next subsequent actuation command to a prescribed fixed interval. The third operative sequence 68 will, based upon the output of the timing means 70, prevent the next subsequent actuation of the second delivery mechanism 48, despite the receipt of a valid medication command, when the elapsed time period is less than the prescribed period.

In the illustrated and preferred embodiment, the third operative sequence 68 also informs the patient through an appropriate "Cannot Administer" message via one or more of the output devices 60/62. In addition, an advisory message can also be transmitted to the central facility 28 via the modem 30. In this way, the system guards against mismedication or overuse of the "on demand" category of medication.

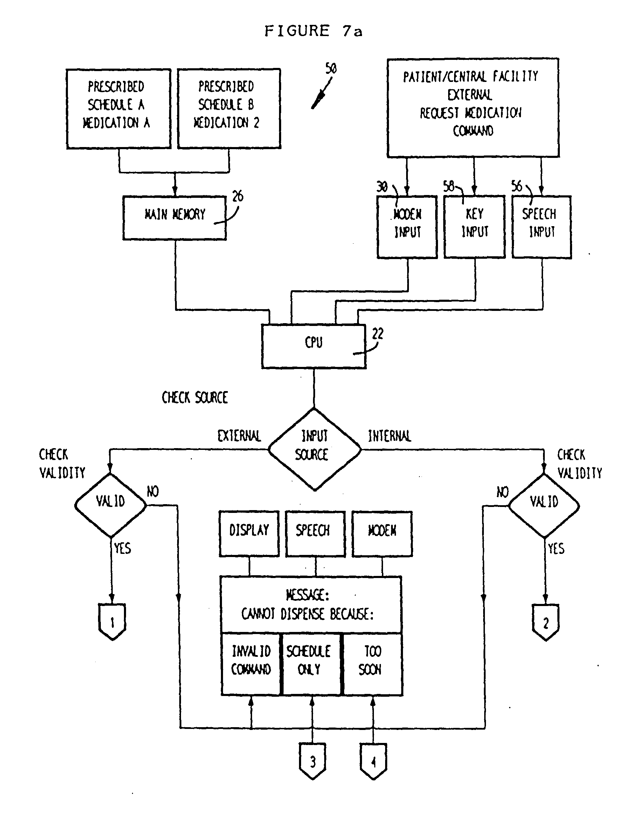

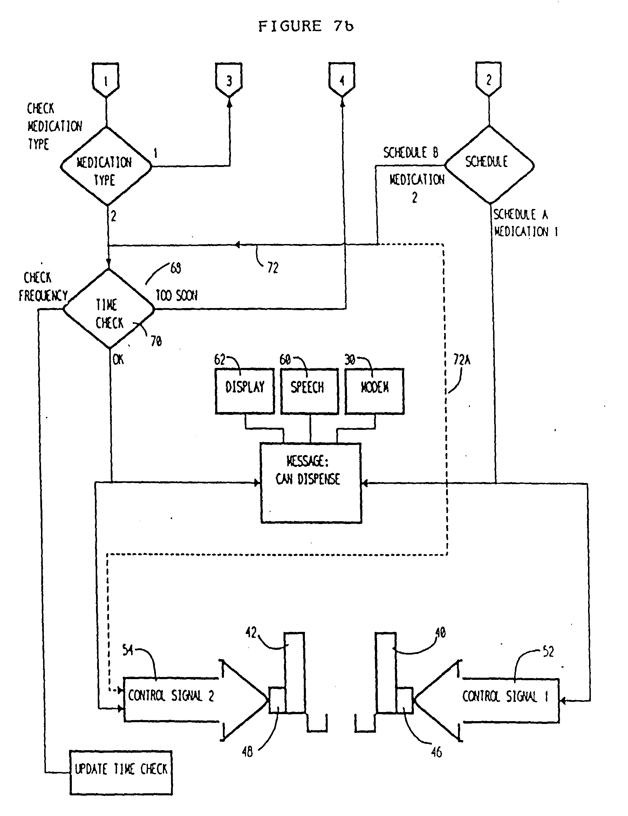

In accordance with another aspect of the invention, the medication delivery system is also adaptable to a situation in which a first category of medication is to be administered only in accordance with a prescribed schedule, while the second category of medication is also administered periodically in accordance with the same or different prescribed schedule, but can also be administered on demand. The control element 50 of this arrangement is shown diagrammatically in Fig. 7. Components shared with the control element 50 shown in Fig. 6 are given the same reference numerals.

In this arrangement, the second operative sequence 66 actuates the second delivery mechanism 42 in response to either a valid internal signal from the prescribed schedule stored in the internal memory 26 (received via preferred path 72 or alternate path 72A in Fig. 7), or in response to the receipt of a valid prescribed medication delivery command from one of the external input devices 30/56/58. Again, timing means 70 is preferably provided (with preferred path 72) to assure that mismedication or overuse does not occur. The prescribed schedule for the second category of medication can be the same as for the first category. Or, as shown in Fig. 7, the different categories of medication have different prescribed schedules.

As before, the second operative sequence 66 also preferably advises the patient, through one or more of the output devices 60/62, upon the release of or the refusal to release the requested medication.

### System for Storing and Administering Medications According to Different Schedules

As just briefly discussed, in another aspect of the invention, the control element 50 is also applicable for use when medication is to be administered according to different prescribed schedules. The control element 50 of this arrangement is also shown diagrammatically in Fig. 7.

In this arrangement, the first and second prescribed medication administration schedules are separately stored in the memory device 26 (designated Schedule A and Schedule B in Fig. 7). The first operative sequence 64 of the control element 50 generates the control signal 52 to actuate the first delivery mechanism 46 in response to a valid signal based upon the first prescribed schedule (Schedule A), but not in response to the second prescribed schedule (Schedule B). The second operative sequence 66 responds to a valid signal based upon the second prescribed schedule (Schedule B) to generate the control signal 54 actuating the second delivery mechanism 48.

### System for Storing and Administering Medication According to Present Health Parameters

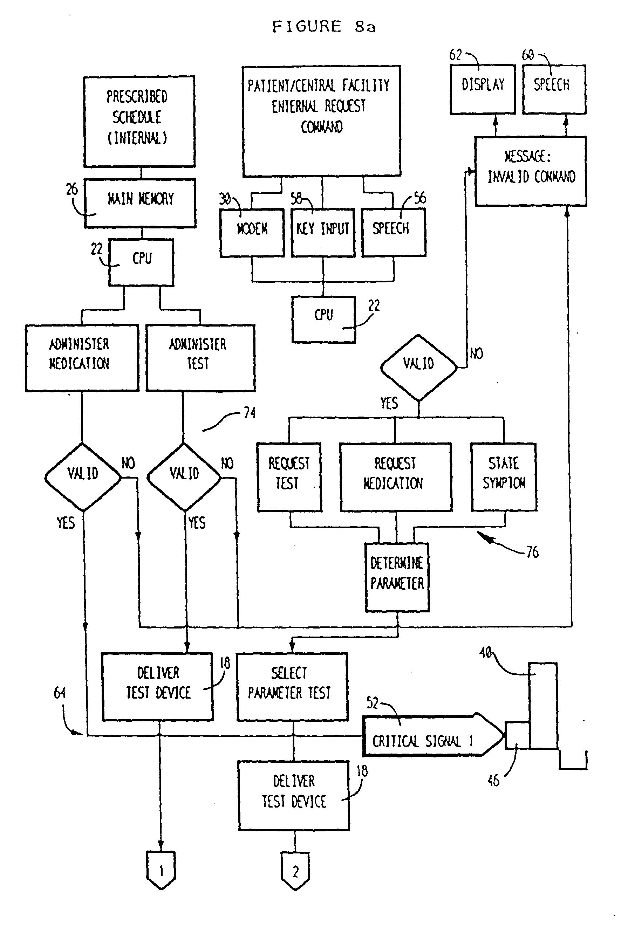

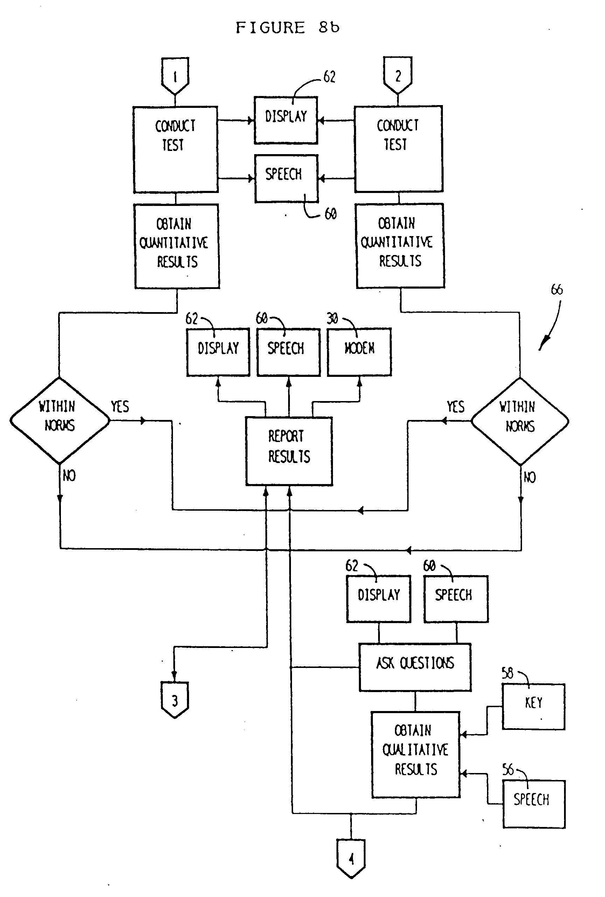

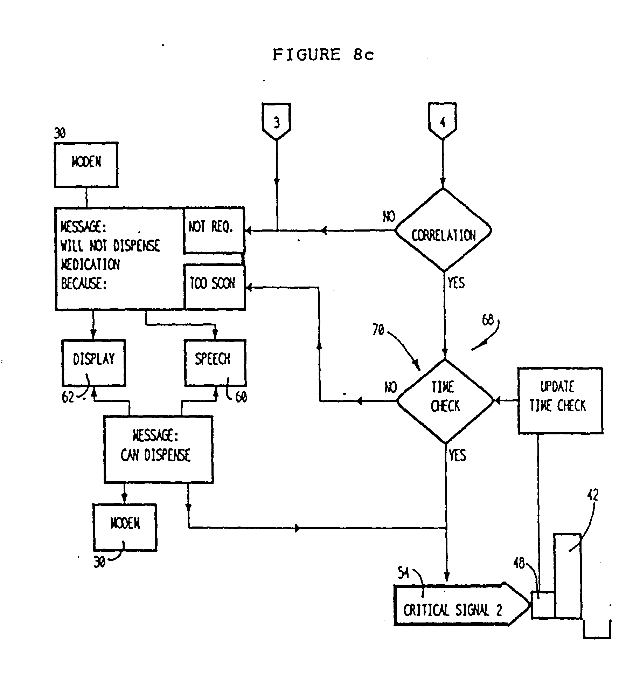

In accordance with yet another aspect of the invention, the control element 50 is also applicable for use when the different input criteria discriminate between one category of medication that can be administered only according to the schedule prescribed by the physician and another category of medication that can or should be administered when the then-existing health parameters of the patient dictate. The control element 50 of this arrangement is shown diagrammatically in Fig. 8. Components shared with the control elements 50 shown in Figs. 6 and 7 are again given the same reference numerals.

In this arrangement, like the arrangements previously described, the prescribed schedule for administering medication is stored in the memory device 26. The first operative sequence 64 responds to this schedule in generating the first control signal 52, as previously described and shown in Fig. 6.

Unlike the other arrangements, however, the second operative sequence 66 shown in Fig. 8 receives and interprets information indicative of selected one or more health parameters of the patient. The second operative sequence 66 compares and correlates this information. If a prescribed correlation exists, indicating an abnormal health parameter that may respond to medication, the second operative sequence 66 generates the second control signal 54 to administer the appropriate medication through the second delivery mechanism 48.

In the illustrated and preferred arrangement shown in Fig. 8, the second operative sequence 66 collects its information from several differing source. One input comprises the quantitative measurements derived from the physiologic testing system 18. Another input comprises qualitative information received from the patient through the external input devices 56/58. Preferably, the second operative sequence 66 affirmatively interacts by prompting the patient, using the external output devices 60/62, to respond by providing qualitative and quantitative information necessary for the second operative sequence 66 to perform its required comparison and correlation sequence.

In the illustrated and preferred arrangement shown in Fig. 8, the second operative sequence 66 itself is prompted into action in one of two ways. In one sequence (path 74 in Fig. 8), an "Administer Test" signal is automatically generated according to a prescribed schedule stored in the main memory 26. This schedule carries out the attending physician's orders to periodically check the vital signs of the patient.

In another sequence (paths 76 in Fig. 8), the "Administer Test" signal is generated in response to an external command issued by the patient or by the central facility 28. For example, the patient can issue a prescribed command by voice (through input 56) or by key input 58 indicating a particular physiologic symptom ("I feel like I have a fever") or a generalized feeling of discomfort ("I don't feel good"). Alternatively, the patient can issue a prescribed command requesting a specific test ("Check my temperature") or request medication ("Give me my pain pills").

The second operative sequence 66 preferably responds initially by requiring further information from the patient through a predetermined sequence of questions designed to positively identify the correct physiologic parameter of concern. These requests are communicated through the output devices 60/62, and the responses are received through the input devices 56/58. Once the source of the physiologic problem is determined, the proper "Administer Test" signal is generated.

However generated, the "Administer Test" signal opens the drawer 38 (as shown in Fig. 2) to make the proper testing device(s) 34/36 available for use by the patient. The "Administer Test" signal also prompts the patient through the output communication devices 60/62 to conduct the desired test or tests.

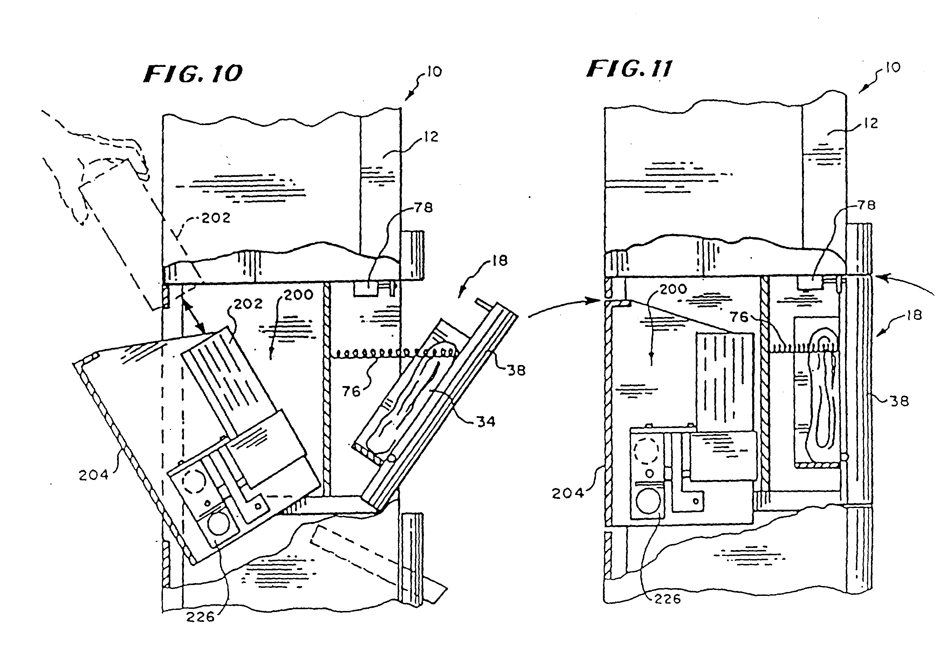

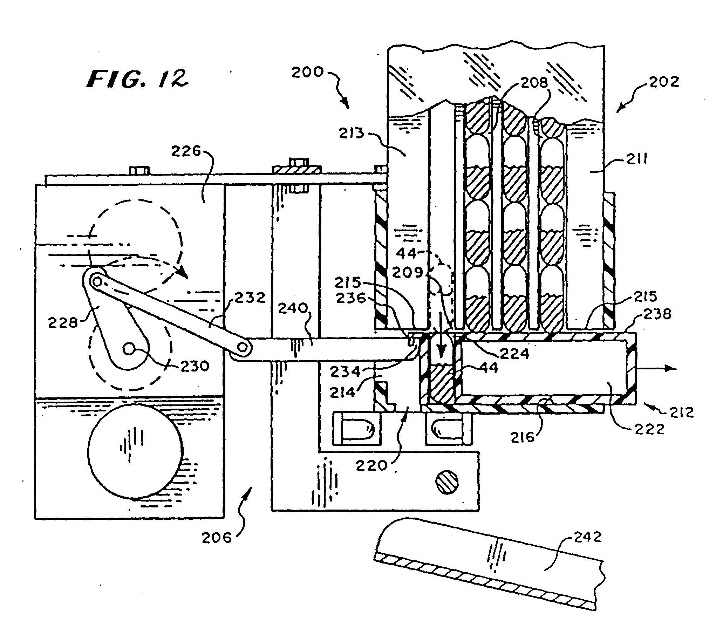

In the illustrated embodiment (see Figs. 10 and 11), the drawer 38 is biased toward an opened position by a control spring 76 (as shown in Fig. 10). A solenoid controlled locking mechanism 78 normally retains the drawer 38 in the closed and locked position (as shown in Fig. 11). An "Open" signal to the solenoid releases the locking mechanism 78 to allow the drawer 38 to open in response to the control spring 76. Other mechanisms can be used to accomplish this or a comparable function.

Referring back to Fig. 8, the quantitative test results obtained by the testing system 18 are compared to prescribed norms. If the test results are within prescribed norms, the second operative sequence 66 issues an appropriate "Will Not Dispense Medication" message through the appropriate output devices 60/62. The normal test results are reported to the patient (via output devices 60/62) and, preferably, to the central facility 28 (via the modem 30) as well.

However, if the quantitative test results are not within prescribed norms, the second operative sequence 66 of the control element 50 proceeds with its further evaluation, taking into account still additional qualitative and quantitative considerations.

For example, should the patient complain of a fever, and the quantitative test results establish an above average temperature, the second operative sequence 66 may additionally prompt the patient, using the external output devices, with a series of questions relating to recent activities, such as exercise or eating, that may effect body temperature. The patient's responses are received and interpreted through the external input devices 56/58.

The second operative sequence 66 of the control element 50 compares and correlates this qualitative and quantitative information in accordance with preprogrammed diagnostic routines. If a correlation exists indicating that the patient has a temperature that is unrelated to recent activities, the second operative sequence 66 generates the second control signal 54 required to activate the second delivery mechanism 48 where the prescribed temperature reducing medication is stored.

In this situation, the second operative sequence 66 also preferable communicates with the patient with an appropriate "Can Dispense Medication" message (via the display 62 and with output speech device 60) and provides an advisory message to the central facility 28 using the modem 30.

In the preferred embodiment, the timing means 70 of the third operative sequence 68 is provided, along with the appropriate advisory message, to assure that symptom-specific medication is not administered too often.

If a correlation does not exist, or if a repeat administration is sought within a given time period, the patient is so informed by an appropriate "Will Not Administer Medication" message, and no medication is administered. However, an advisory message may nevertheless be transmitted to the central facility 28 or to a designated family member using the modem 30.

As shown in Fig. 3, the medication delivery system can separately store several different types of medication to treat different physiologic symptoms, such as high temperature, indigestion, or body aches. The control element 50 can receive and correlate the qualitative and quantitative information, and, upon arriving at a prescribed correlation, generate the specific control signal to administer the appropriate type of medication, depending upon the symptom encountered.

### System for Altering Prescribed Medication Schedules For Remote Administration

In accordance with yet another aspect of the invention, the control element 50 can further include means for temporarily altering the routine dictated by a prescribed schedule stored in the internal memory, or for permanently changing the medication administration schedule, in response to the receipt of a prescribed command from either the patient or the central facility 28.

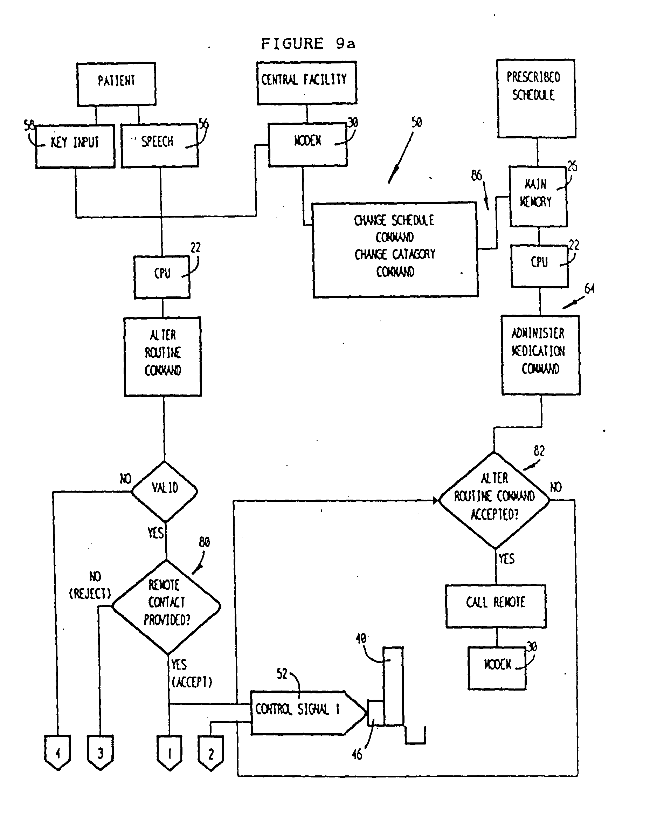

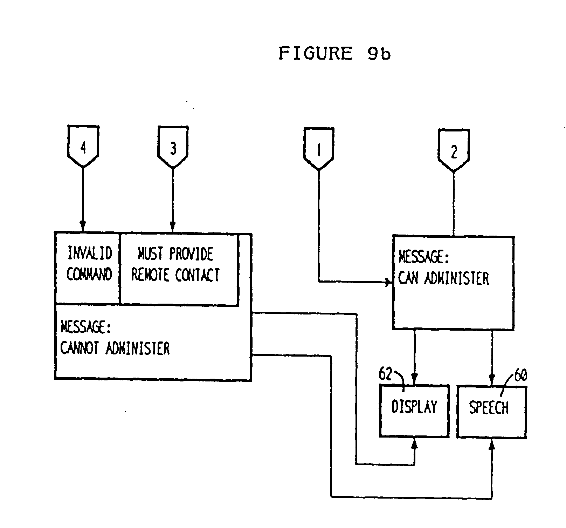

A control element 50 that serves this purpose is shown diagrammatically in Fig. 9. As before described, the control element 50 includes a first operative sequence 64, which bears the same reference numeral assigned before. The first operative sequence 64 administers medication in accordance with an internal signal generated according to a prescribed schedule contained in the main memory 26.

In this embodiment, the control element 50 comprises a fourth operative sequence 80 that alters the-prescribed routine upon response to a prescribed "Alter Routine" command. The specific arrangement shown in Fig. 9 is based upon a representative situation where the patient will be away from home when a prescribed medication administration event is scheduled to occur. In this situation, the fourth operative sequence 80 serves to administer the medication in advance of the schedule, so that the patient can take the medication with him/her for administration at the prescribed time. To assure compliance, the fourth operative sequence 80 also serves to affirmatively contact the patient at his/her away-from-home location when it is time to take the medication.

In this situation, the prescribed "Alter Routine" command may be issued either by the patient or by the central monitoring facility 28, according to the procedure prescribed by the attending physician. Upon receipt of a valid "Alter Routine" command, the fourth operative sequence 80 will require the patient to provide a phone number where he/she can be reached at the time the next prescribed medication event occurs. The fourth operative sequence 80 will release the medication only if a remote contact point is provided by the patient.

In this arrangement, the fourth operative sequence 80 establishes a check point 82 within the routine of the first operative sequence 64. The check point 82 determines whether a valid "Alter Routine" command has been accepted by the fourth operative sequence 80. If it has, the first operative sequence 64 will respond to the prescribed schedule by generating a signal (via path 84 in Fig. 9), not to administer medication, but to contact the patient at the remote contact point using the modem 30. A message is transmitted to remind the patient that the prescribed time for taking the medication has arrived.

In one arrangement, the fourth operative sequence 80 can communicate with the away-from-home patient through a paging or cellular telephone system. Likewise, the patient can remotely communicate with the device when away from home.

In the "Alter Routine" sequence illustrated in Fig. 9, the fourth operative sequence 80 of the control element 50 does not alter the memory resident schedule itself. The prescribed schedule remains the same and controls during the next prescribed medication event, unless another "Alter Routine" command is accepted.

In the embodiment illustrated in Fig. 9, the control element includes a fifth operative sequence 86 to actually change the memory resident schedule in response to a valid "Change Schedule" command.

The specific arrangement shown in Fig. 9 is based upon a representative situation where the attending physician has changed the drug regime. The central monitoring facility 28 remotely issues a "Change Schedule" command to gain access to and alter the memory resident schedule. Preferable, a "Change Schedule" command will be received and interpreted by the system only with appropriate pass word precautions and safeguards.

In the same manner, the attending physician can by issuing a prescribed "Change Category" command to change the administration criteria of medication retained by the system. For example, the physicians can remotely make a medication that could be administered "on demand" to a "schedule only" category, and vice versa.

### A PREFERRED PHYSICAL EMBODIMENT

The specific configuration of the interactive medication delivery system 20 as above described can vary according to the form in which the medication is administered. For example, one or more types of medication can be administered in predetermined dosages in sealed packets (commonly called "blister packs"). Alternatively, or in combination, single dosages of a medication can be administered in a pill or caplet form, either in unsealed, "loose" form or on sealed rolls.

Attention is now directed to Figs. 1 to 3 and 10 to 15, where a system 200 for storing and delivering individual pills or caplets is shown that embodies the features of the invention.

The system 200, as the previously described system 100, is carried within the confines of an overall patient monitoring and assistance device 10 (see Figs. 1 to 3). The system 200, just as the one previously described, also includes discrete medication storage compartments 202 A through J (see Fig. 3). The storage compartments 202 are each capable of separately storing medication in pill or caplet form.

In the illustrated embodiment shown in Fig. 3, there are ten storage compartments 202 located within an enclosed housing. Of course, the number of individual compartments can vary according to the needs of the patient. Each compartment 202 is capable of holding a number of individual pills/caplets (designated by reference numeral 44), depending upon their size. As shown in Fig. 3, the pills/caplets 44 are arranged side-by-side in a plurality of vertically stacked columns within each compartment 202. As shown in Fig. 3, the compartments 202 can differ in vertical height and/or transverse thickness depending upon the size of the pills/caplets housed therein. The differing size of the compartments 202 (particularly in terms of thickness) also assures the proper ordered arrangement of the compartments 202 within the system 200.

The housing 204 that encloses the compartments 202 is mounted in the device 10, behind the drawer 38 that contains the testing devices (see Figs. 10 and 11). The housing 204 can be tilted out from back of the device 10 for service and to load medication into the system 200 (see Fig. 2 also).

In the illustrated embodiment, as best seen in Fig. 3, the system 200 includes ten separate medication delivery means or mechanisms 206 A through J, one associated with each storage compartment 202. Each mechanism 206 is individually controlled by one of the control elements 50 in response to a prescribed control signal or signals in the manner previously described.

Each delivery mechanism 206 is identical in construction, so only one will be described in detail.

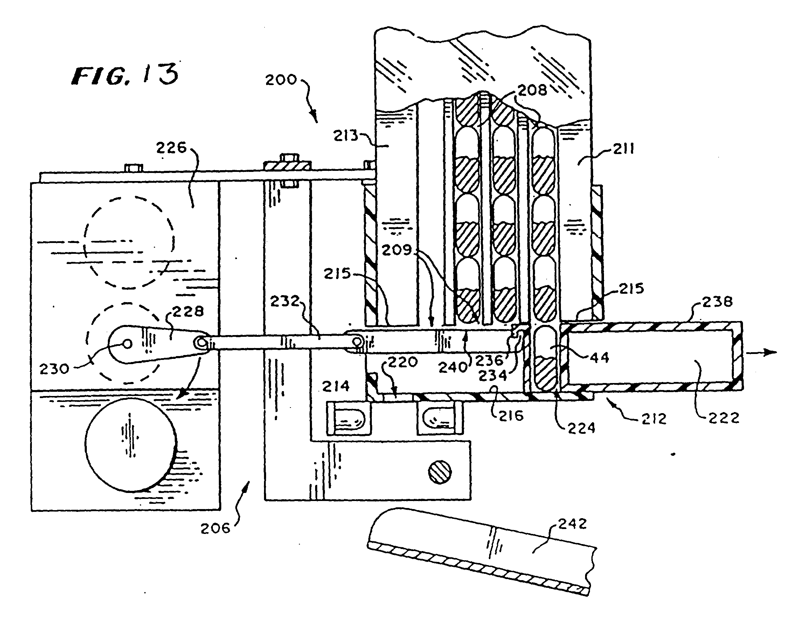

In the illustrated arrangement (as best shown in Figs. 12 and 13), the bottom of each vertical medication storage column 208 opens into a channel 210 that spans the bottom of the compartment 202. The channel 210 includes an open front end 212 and an open back end 214 (respectively positioned to the right and to the left in Figs. 12 and 13). The channel 210 also includes a bottom wall 216 and two upstanding sidewalls (not shown in the side sectional view). The channel bottom wall 216 includes a opening 220 adjacent its open back (left) end 214.

The delivery mechanism includes a shuttle member 222 that is movable within the channel 210 between a rearward position, fully within the channel 210 (shown in Fig. 12), and a forward position, extending partially outside the open front end 212 of the channel 210 (shown in Figs. 3 and 13). The shuttle member 222 includes an open passageway 224 that registers with the bottom opening 220 in the channel 210 when the shuttle member 222 is in its rearward position (see Fig. 12). Movement of the shuttle member 222 successively toward the forward position brings the passageway 224 into sequential registration with the open bottom of each of the storage columns 208.

A linkage assembly couples each shuttle member 222 to an associated electric motor 226 to drive the shuttle member 222 laterally forward and backward within the channel 210. While the construction of the linkage assembly may vary, in the illustrated embodiment (see Figs. 12 and 13), it includes a rotating crank 228 coupled to the drive shaft 230 of the associated motor 226. A double pivoted link 232 is attached at one end to the crank 228. The other end of the double pivoted link 232 includes a hook 234 that attaches to a lip 236 on the end wall of the shuttle member 222.

Rotation of crank 228 thereby imparts forward and rearward pivotal movement to the shuttle member 222. In particular, as shown in Figs. 12 and 13, one full revolution (360-degrees) of the crank 228 will cycle the shuttle member 222 from its rearward position (Fig. 12) into its forward position (Fig. 13) and back.

As the shuttle member 222 is moved out toward its forward position (see Fig. 12), the passageway 224 will successively come into and out of registry with the bottom of each storage column 208 beginning with the rearwardmost (farthest left) column. The first bottommost pill/caplet encountered in a column will fall by gravity into the empty passageway 224. The closed bottom 216 of the channel 210 retains the fallen pill/caplet within the passageway 224 as the shuttle member 222 moves into is fully forward position and back toward its rearward position. The presence of the retained pill/caplet prevents another pill/caplet from falling into the passageway 224. Likewise, the leading top wall portion 238 of the shuttle member 222 and the trailing top portion 240 of the link 232 serve to progressively close the bottoms of the other columns as the shuttle member 222 is advanced, preventing additional pills/caplets from entering the channel 210.

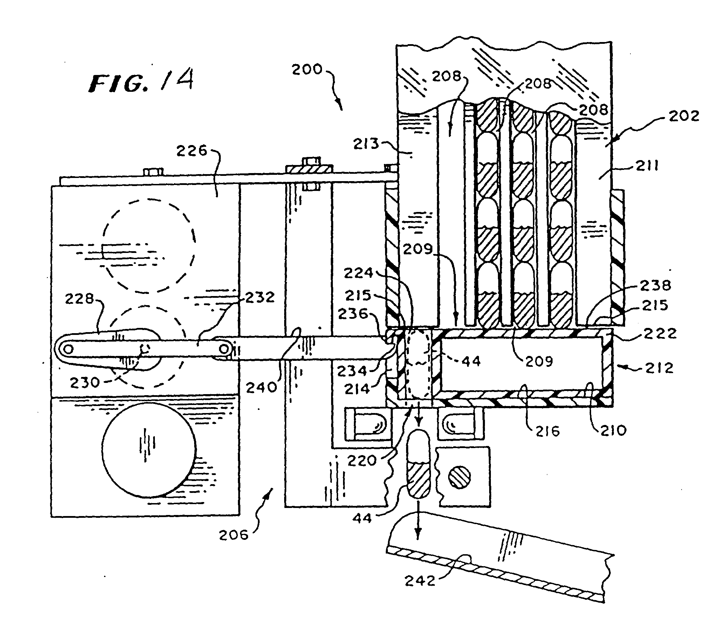

When the shuttle member 222 returns to the rearward position (see Fig. 14), the passageway 224 will again register with the bottom channel opening 220. The retained pill/caplet will fall from the passageway 224 through the bottom channel opening 220 and then into a delivery chute 242 that leads to a medication dispenser 244 at the front of the device 10 (see Fig. 3 also).

As shown in Figs. 1 and 2, the medication dispenser 244 is movable between a closed position (Fig. 1) and an opened position (Fig. 2). A spring 245 (see Fig. 3) normally biases the dispenser 244 toward the opened position, and a solenoid controlled latching mechanism 247 is provided to lock the dispenser 244 in the closed position. At the time medication is released into the delivery chute 242, the dispenser 244 is located in its locked and closed position. Upon delivery of the medication to the dispenser 244, a signal to the latching mechanism 247 allows the dispenser 244 to move into its opened position in response to the bias of the spring 245. The dispensed medication is thereby made available to the patient. Upon taking the medication, the patient closes the dispenser 244, preferably in response to a prompt generated by the device 200.

As in the previously described embodiment, in the illustrated and preferred embodiment of this system (see Fig. 10), each compartment 202 can be individually removed from the housing 204 as a module for replenishment of the medication when the housing 204 is tilted through the back of the device 10. The removable, interchangeable modular design of the compartments 202 simplifies a change in medication brought about by a change in the prescribed medication regime.

Again, it is contemplated that the modular compartments 202 will be prepacked by trained medical or pharmacy personnel at a location away from the device 10 and then carried on site.

In the illustrated arrangement shown in Fig. 10, the motors and linkage assemblies remain in the housing 204 upon removal of the compartments 202. The hooked end 234 of the pivoted link 232 is easily engaged and disengaged from the lip 236 of the associated shuttle member 222.

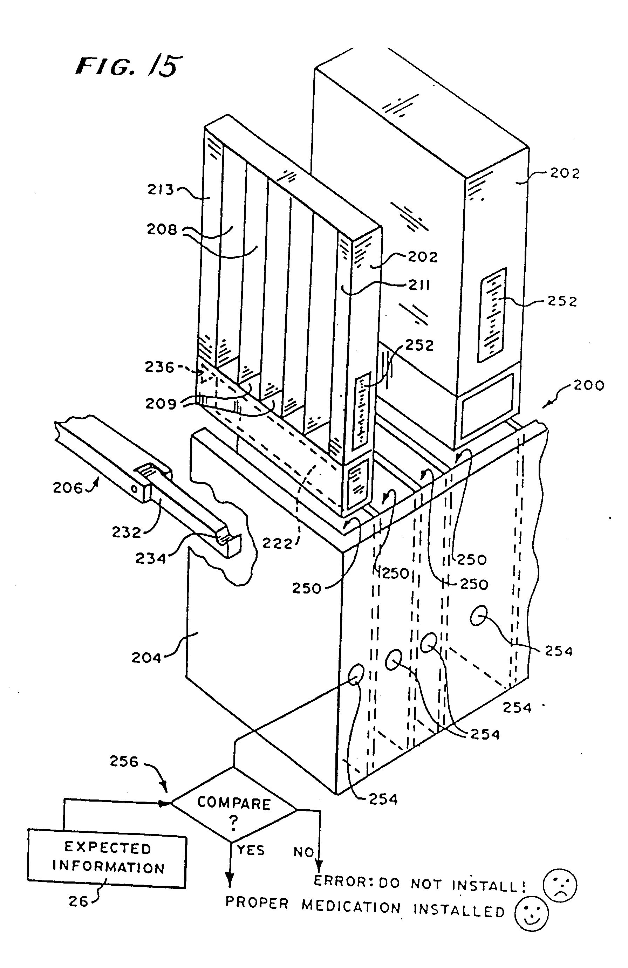

The housing 204 includes slots 250 arranged to receive and retain the compartments 202 in proper alignment with the associated linkage assembly (see Fig. 15). The slots 250, like the compartments 203, differ in size, so that a given compartment 202 will uniquely physically fit into only a selected one of the slots 250. This assures the desired ordered arrangement of medication within the dispensing system 200.

In the illustrated and preferred embodiment (see Fig. 15), each compartment 202 is uniquely identified using machine readable code 252. In the illustrated embodiment, the code 252 is readable by an optical scanning system 254 associated with each slot. The code 252 contains information about the medication carried within the associated compartment 202, such as the type of medication, the number of dosages contained, and the patient's name or prescription number. The scanning system 254 reads the code 252 as the compartment 202 is inserted into the appropriate fitting slot 250.

As shown in Fig. 15, the control element 50 for the dispensing system 200 preferably includes a comparator 256 that compares the information sensed by the scanning system 254 with an expected result carried in the main memory 26. If the sensed information is not the expected result - for example, when the medication for the wrong patient is being accidentally loaded into the system 200 - an appropriate error message is generated.

The system 200 thus assures, in fail-safe fashion, the placement of the prescribed medication for administration to the patient.

The system 200 shown in Figs. 1 to 3 and 10 to 15 includes the separate medication compartments and ten individually controllable delivery mechanisms, one for each compartment. The system 200 can include any one of the control elements 50 shown in Figs. 6 to 9. The selected control element 50 serves to individually activate the motors 226 associated with each of the compartments 202 by generating different control signals in response to different input criteria in the manner previously described.

For example, if a medication regime requires the administration of three different pills/caplets according to a prescribed schedule, the control element 50 associated with the system 200 can simultaneously generate a first control signal to each of the delivery mechanisms associated with the particular compartments in which the prescribed pills/caplets are located. The three pills/caplets would therefore be dispensed at the same time (as shown in Fig. 3). The control element 50 could dispense other pills/caplets according to different prescribed schedules, or upon patient demands, upon the issuance of appropriate control signals to the other delivery mechanisms.

The system 200 shown in Figs. 1 to 3 and 10 to 15 is thereby capable of storing and coordinating the administration of up to ten different categories of medication in pill or caplet form in accordance with one or more prescribed schedules, upon demand, or upon any other selected administration criteria.

### ASSOCIATED SYSTEM FOR MAINTAINING AND MANAGING A MEDICATION INVENTORY

In accordance with another aspect of the invention, the medication delivery system 20 as above described forms a part of an overall system 100 that inventories and otherwise manages the medication regime of a patient. This system 100 is shown in Fig. 16. As there shown, the inventory and management system 100 can be maintained in the mass storage system at the central monitoring facility 28, or within on-site internal memory 26, or (preferably) at both locations.

The inventory and management system 100 can network the central monitoring facility 28 with the medical delivery systems (designated 20A, 20B, and 20C in Fig 16) of a number of patients, thereby centralizing inventory and management control over the medication regimes of different patients.

The inventory management system 100 maintains a log 102A/B/C for each medication delivery system 20A/B/C in the network. Each log 102A/B/C is preferably maintained on site by each delivery system 20A/B/C as well as at the central monitoring facility. While the contents of each log 102A/B/C will differ, the basic format of each is the same. Therefore, only one (log 102A) will be described.

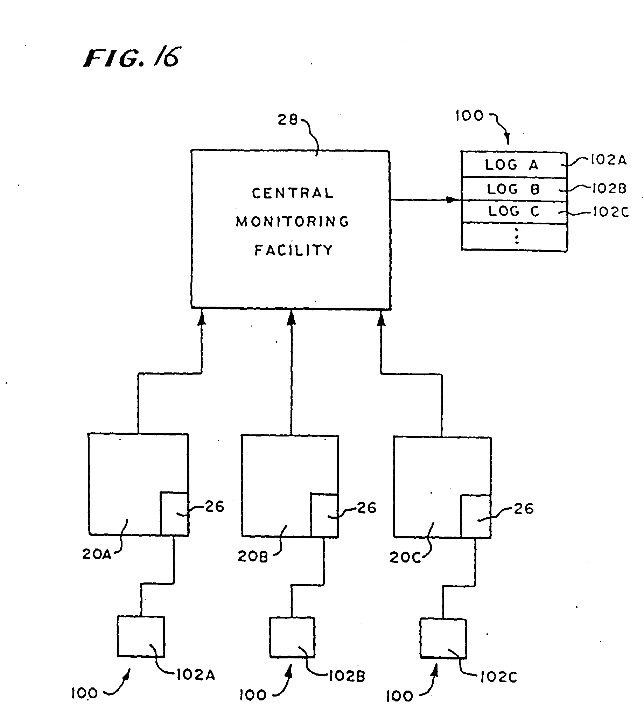

As shown in Fig. 17, the log 102 includes a separate data file for each medication storage compartment in the associated system 20, either in sequential or random access format. In Fig. 17, two medication storage compartments 40 and 42 are shown for illustration purposes (as in Fig. 7), and there are thus two associated data files 104 and 106. Each file 104 and 106 includes a number of records, which contains the information necessary to oversee and manage the administration of the particular medication stored in the associated compartment. These records are generally identified in Fig. 17 as R1, R2, R3, etc.

In the illustrated embodiment, each medication file 104 and 106 includes a first record (R1) 108 that reflects the date and time the medication was administered according to the schedule in internal memory 26; a second record (R2) 110 that reflects the date and time the medication was administered on demand; and a third record (R3) 112 that reflects the date and time the system 20 refused to administer medication.

Each medication file 104 and 106 also includes a fourth record (R4) 114 that contains a number that represents the amount of the medication presently remaining within the associated storage compartment 40/42.

Each medication file 104 and 106 can include a number of additional records depending upon the level of detail required. For example, the medication file can include a record that reflects the category of the medication as programmed in the control element 50 (by schedule, on demand, or both); the minimum dosage interval programmed in the timing means 70 (see Fig. 8); the prescribed dosage in terms of time and amount programmed in internal memory 26; the storage compartment number where the medication is located within the delivery system 20; and the date on which the storage compartment was last replenished.

The first, second, third, and fourth records 108/110/112/114 are created and updated based upon signals generated by the associated control element 50. In Fig. 17, the control element 50 shown in Fig. 7 is contemplated.

The generation of the first control signal 52 and accompanying "Can Administer" message loads date/time data into and updates the first record 108 of both files 104 and 106 (it is assumed that the medications contained in compartments 40 and 42 are each administered according to the schedule).

The generation of the second control signal 54 and accompanying "Can Administer" message loads date/time data into and updates the second record 110 of the second file 106 (it is assumed that only the medication contained in the second compartment 42 can be administered upon demand).

The generation of a "Cannot Administer" message loads date/time data into and updates the third record 112 of each file 104/106.

The fourth record 114 of each file 104/106 is initialized each time the medication in the associated storage compartment 40/42 is replenished on site, to thereby represent the amount of medication initially placed inside the compartment 40/42. In the illustrated embodiment, it is contemplated that the person who replenishes the medication on-site will manually initialize the fourth record 114 through an appropriate entry made using the input keys 58. Of course, the initialization can be manually made, via the modem 30, from the central facility 28. The initialization could also be accomplished automatically when the compartment 40/42 is loaded into the system 20, by using the machine readable code and associated scanning device previously described.

Once initialized, the fourth record 114 of each file 104/106 is updated by reducing the initialized amount by one whenever a first or second control signal 52 and 54 is generated, as appropriate.

In the illustrated and preferred embodiment (see Fig. 17), the system 100 also includes a comparator 116 associated with each medication file 104/106. Each time the fourth record 114 is updated and/or at prescribed periodic time intervals, the comparator 116 compares the number stored in the fourth record 114 with a preselected number stored in the main memory 26 that represents a minimum storage amount for that particular storage compartment. When the number stored in the fourth record 114 is equal to or less than the associated minimum storage amount, a "Replenish" signal is generated to alert the patient and the central monitoring facility 28. A person is then sent to replenish the medication contained in the associated compartment 40/42 and to initialize the fourth record 114.

The system 100 thus continuously monitors the patient's medication regime and assures that medication sufficient to comply with the regime is available within the medication delivery system 20.

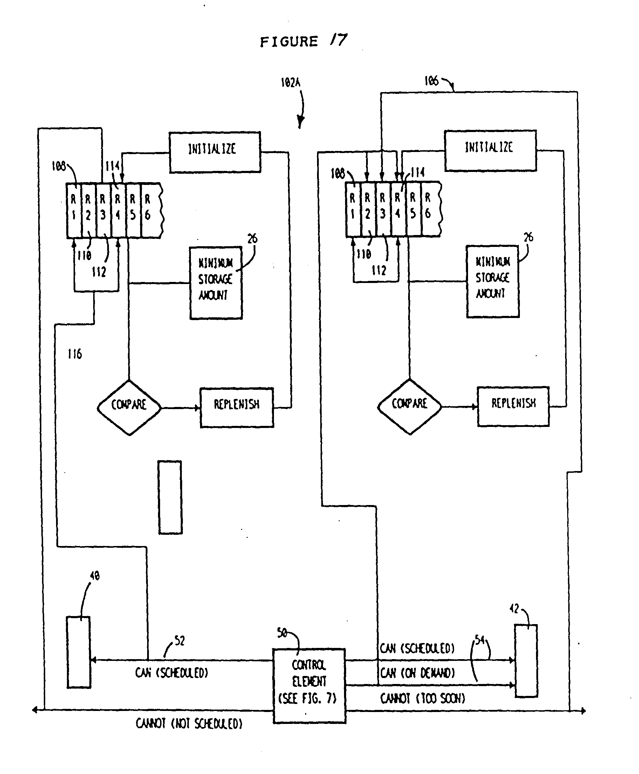

As shown in Fig. 18, the system 100 preferably also includes a follow up sequence 118 for determining whether medication that is dispensed is actually taken by the patient. In the illustrated embodiment, the follow up sequence 118 comprises a loop 120 that, after the medication is dispensed, inquires whether the dispensed medication was taken. The inquiry is made either through the speech output device 60 or the display 62, or both. The loop 120 will periodically repeat the inquiry unless either an affirmative or negative response is provided by the patient, either by a voice command or through the input keys.

If an affirmative response is received, the appropriate first or second record 108/110 is updated to reflect that the medication dispensed was actually taken.

If a negative response is received, the follow up sequence 118 will first determine whether the patient accidentally lost the dispensed medication through an additional series of inquiries by voice and/or display.

The follow up sequence may inquire "Did you lose the medication?" If the patient's response (by voice or by input key) indicates that the medication was dropped and lost, the appropriate first and second record 108/112 is updated to reflect this fact. In this instance, the central facility 28 is alerted. The central facility 28 may then investigate and issue the appropriate "Repeat Administration" command to administer a repeat dose of the lost medication. The "Repeat Administration" command overrides any associated timing means 80.

If the patient will not respond, the follow up sequence 118 may warn the patient, "If you do not take your medication within three minutes, I shall notify the central facility" and wait three minutes for an affirmative response. If the affirmative response is not received, the appropriate first or second record 108/110 is updated accordingly. The central facility 28 is also alerted via the modem 30 that the patient has failed to take the dispensed medication. The central facility 28 will then investigate.

### SYSTEM FOR REMOTE VISUAL MONITORING

In an illustrated and preferred embodiment shown in Figs. 19 to 23, the device 10 and central monitoring facility 28 are further linked by a system 300 that visually monitors the patient's activities and physiologic condition.

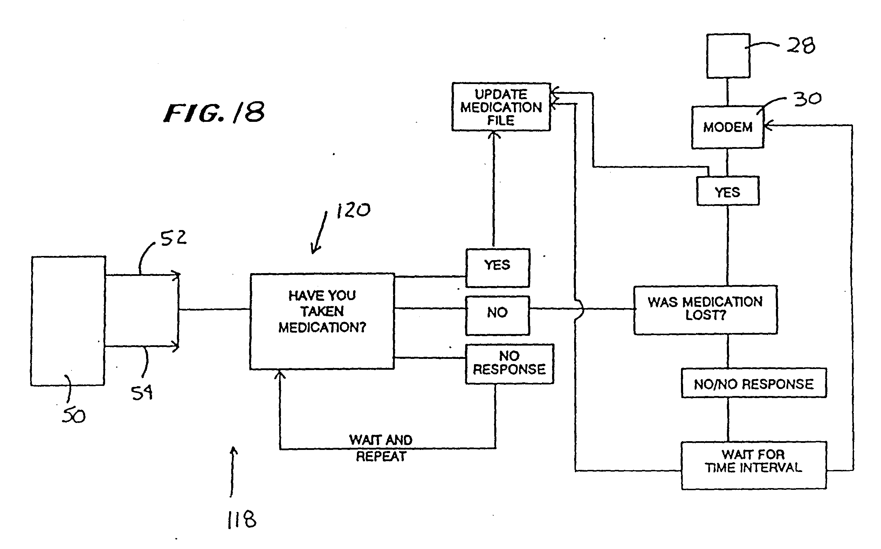

The monitoring system 300 includes a module 302 (see Figs. 19 and 20) mounted on top of the cabinet 12. The module 302 includes a control cable 304 that electrically links the module 300 to the main CPU 22 of the device 10 (see Fig. 29).

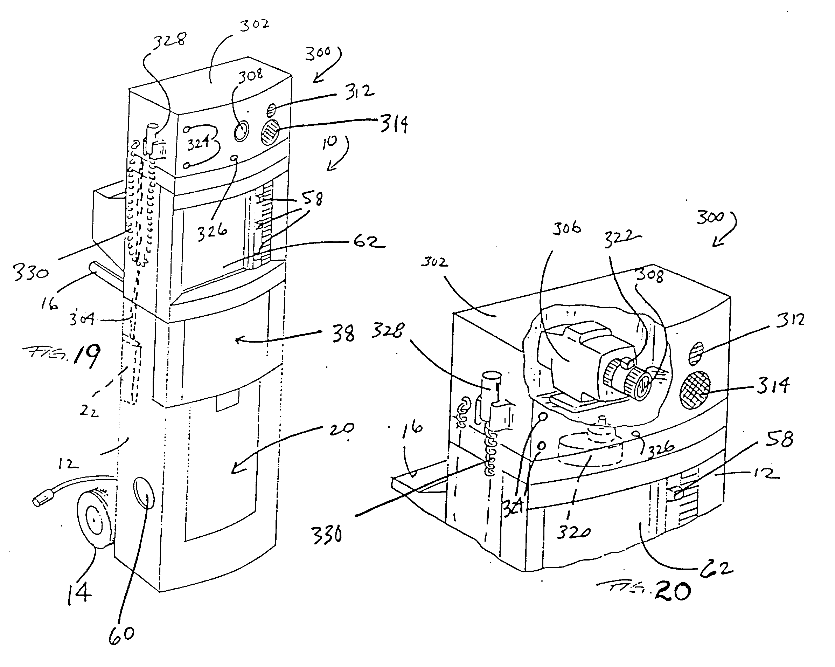

The module 302 carries within it an observation camera 306 (see Fig. 21). The observation camera 306 takes the form of a conventional video camera with a wide angle lens 308. The lens 308 provides a viewing angle and depth of field sufficient to include the width and depth of the room where the device 10 is installed.

The observation camera 306 has the sensitivity to work under normal and typical "low light" indoor conditions. The observation camera 306, when actuated, records video images of the patient and the surrounding environment.

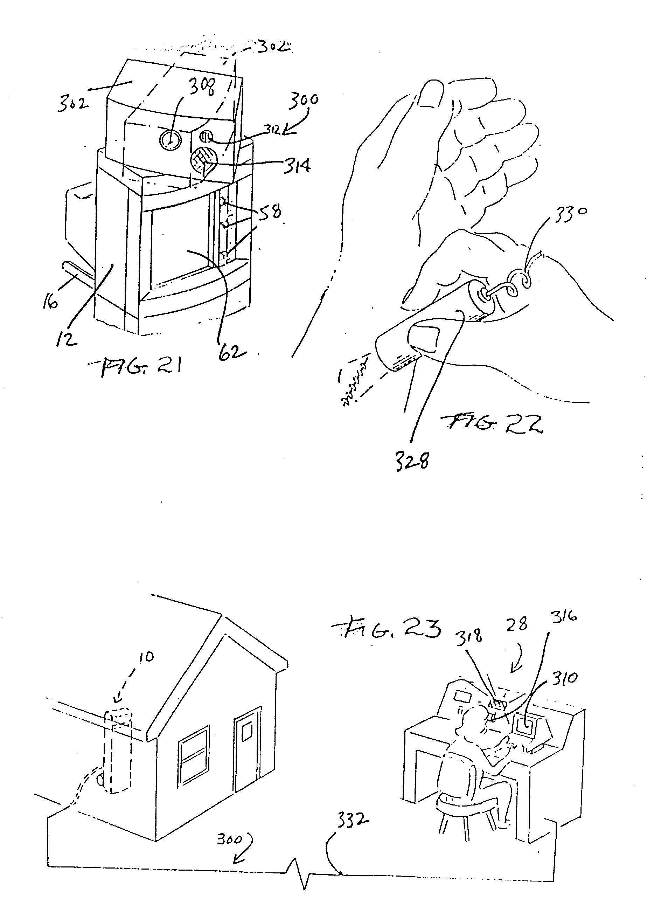

The video signals of the observation camera 306 are digitalized by the main CPU 22 for transmission to the central facility 28 (see Fig. 23). The digitalized video signals are transmitted by the CPU 22 to the central facility 28 using conventional twisted copper lines 332, by fiber optic technology, or other cellular, cable, or satellite transmission device.

The monitoring system 300 further includes a CRT monitor 310 installed at the central facility 28. The CRT monitor 310 receives the video signals transmitted by the observation camera 306. Personnel at the facility can thereby visually monitor the patient's movement, physical appearance, and demeanor.

If desired, another CRT monitor (not shown) linked to the observation camera 306 can be installed at the home of a family member of the patient, or another designated caregiver. This person can use the system 300 to monitor the patient's well-being, too.

The system 300 also preferably provides an audio link between the device 10 and the central facility 28 to accompany the visual link the observation camera 306 provides. The audio link comprises a microphone 312 and speaker 314 on the module 302 (see Fig. 19) for use by the patient. It also includes a microphone 316 and speaker 318 for use by central facility personnel. The system 300 thereby provides two-way, interactive visual and audio communications between the patient and the remote facility 28.

The CPU 22 can also store in memory the digitalized video images and audio signals of the observation camera 306 and microphones 312/316. The system 300 thereby serves to create a historical visual and audio record to augment and complement the other record keeping functions of the patient management system 100, already described.

To supplement the viewing function, the system 300 provides a motor driven swivel mount 320 for the module 302 (see Fig. 20). The swivel mount 320 can be remotely operated by personnel at the central facility 28 to rotate the module 302 and, with it, the observation camera 306 atop the housing 12 (see Fig. 21). In this way, the observation camera 306 can scan the room where the device 10 sits to locate the patient and obtain the best view.

The system 300 also preferably includes a motorized zoom mechanism 322 for the wide angle lens 308 (see Fig. 20). The zoom mechanism 322 can also remotely activated by personnel at the central facility 28 to adjust the focal plane of the observation camera 306 to best view the patient.

The monitoring system 300 also preferably includes the patient to operate the swivel mount 320 and the zoom lens mechanism 322.

In the illustrated and preferred embodiment, the swivel mount 320 and zoom mechanism 322 is responsive to the sound of the patient's voice. Using voice commands, the patient can operate the swivel mount 320 to swing the observation camera 306 around. Using voice commands, the patient can also operate the zoom lens mechanism 322. For example, by saying "Closer" or "Farther," "Left" or "Right," the patient can adjust the lens 308 and move the camera 306 to help the central facility 28 obtain the best picture for viewing.

In an alternative embodiment, the system 300 includes conventional sensors 324 on the module 302 that sense movement, or to body heat, or both. The sensors 324 are linked to the CPU 22 to actuate the swivel mount 320 and lens mechanism 322 to operate the observation camera 306 and actively search out the location of the patient, in the absence of particular sound or voice command. The central facility 28 can remotely actuate these sensing features of the monitoring system 300 when necessary to seek out the patient.

The operation of the observation camera 306 to record and store visual images can be carried out in various ways.

The central facility 28 can turn on the observation camera 306 at any time upon a prescribed remote actuation command sent to the CPU 22.

The patient, too, can actuate the observation camera 306 at any time by a prescribed local command to contact the central facility 28. Preferably, the patient uses a prescribed verbal command to accomplish this.

The command to actuate the observation camera 306 can also coincide with other commands that actuate other functions of the device 10. For example, an "Administer Test" command (path 76 in Fig. 8a) can also actuate the observation camera 306, when the patient says "I don't feel good." Or, the observation camera 306 can be actuated whenever the patient requests medication at an unscheduled time.

The observation camera 306 can also be actuated according to a prescribed time schedule. The time schedule can coincide with a scheduled medication or physiologic testing event, or otherwise. In this way, the central facility can observe the patient while medication is being administered, or while the patient tests a physiologic parameter.

Alternatively, or in addition, the observation camera 306 can be actuated at prescheduled intervals between prescribed medication or testing periods. In this way, the central facility 28 can periodically check upon the patient.

Alternatively, or in addition, the observation camera 306 can be actuated during the medication follow up sequence 118 earlier described, or in connection with any other function the system 100 performs.

The observation camera 306 can also be actuated by the control element 50 itself, independent of either the patient or central facility 28, in response one prescribed input or series of inputs received by the medication delivery system 20 during the course of operation.

For example, if the control element 50 receives input or a series of inputs that indicate the presence of a abnormal health parameter, the control element 50 itself can actuate the observation camera 306, while it also alerts the central facility 28. Thus, if the control element 50 senses quantitative test results (obtained by the testing device(s) 34/36) that establish a steady rise or fall in the patient's temperature or blood pressure, or like changes in physiologic parameters, the control element 50 can alert the central facility and turn on the observation camera 306.

Alternatively, or in addition, the monitoring system 300 can include a sound sensor 326. The control element 50 senses receipt of signals by the sensor 326, indicating that the patient is up and about. When the sound sensor 326 fails to receive signals over a predetermined period, the control element 50 can turn on the observation camera 306 and alert the central facility 28.

Thus, should the patient lose consciousness or otherwise become incapacitated, the lack of patient activity will in time be noticed by the control element 50. Under these circumstances, the control element 50 alerts the central facility and actuates the observation camera 306 to check up on the patient's well being.

The monitoring system 300 also preferably includes a diagnostic video camera 328 that provides close-up, high resolution video images. The diagnostic camera 328 is preferable a conventional small, handheld mini-camera that can be operated by the patient or by a designated caregiver for the patient. In this arrangement, the camera 328 is electrically attached to the module 302 by flex cable 330.

The diagnostic camera 328 allows personnel at the central facility 28 to view close-up, localized areas of the patient's body (see Fig. 22). These close, localized views assist the central facility personnel in remotely assessing and investigate patient complaints of discomfort, pain, or trauma. The close, localized views that the camera 328 provides also allow central facility personnel to remotely perform routine post-surgical inspection of incisions and catheter sites. The camera 328 can also allow central facility personnel to carry out prescribed wound management and measurement tasks remotely.

Like the observation camera 306, the central facility 28 can actuate the diagnostic camera 328 at any time upon a prescribed remote actuation command sent to the CPU 22. The patient, too, can actuate the diagnostic camera 328 by a prescribed local, and preferably verbal, command.

Like the observation camera 306, the digitalized video images of the diagnostic camera 328 can be stored by the CPU 22, if desired. The diagnostic camera 328, like the observation camera 306, can also serve to create a historical visual record to augment and complement the other diverse record keeping functions that the patient management system 100 provides.

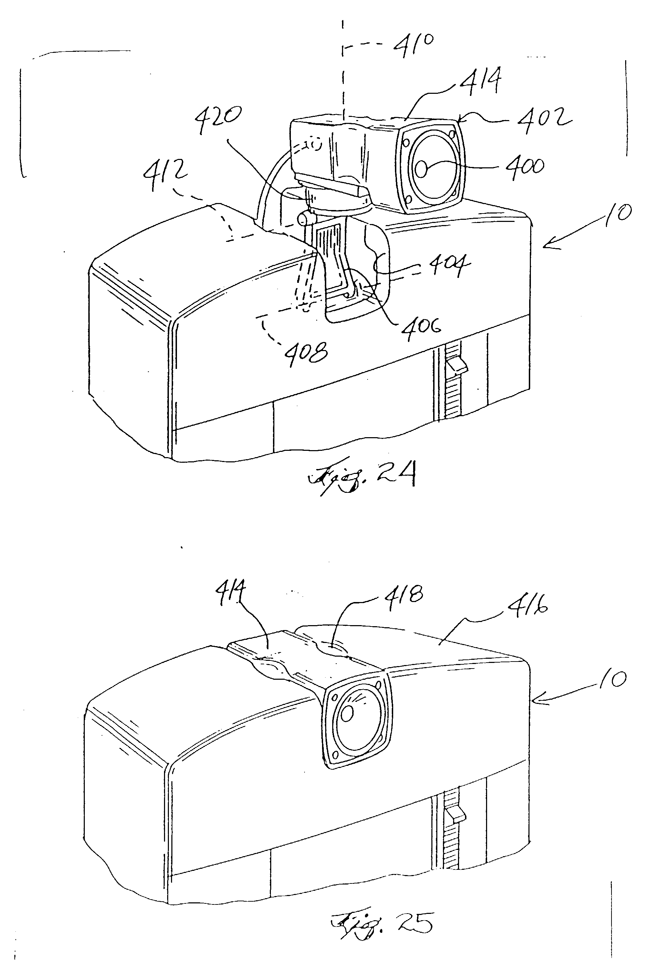

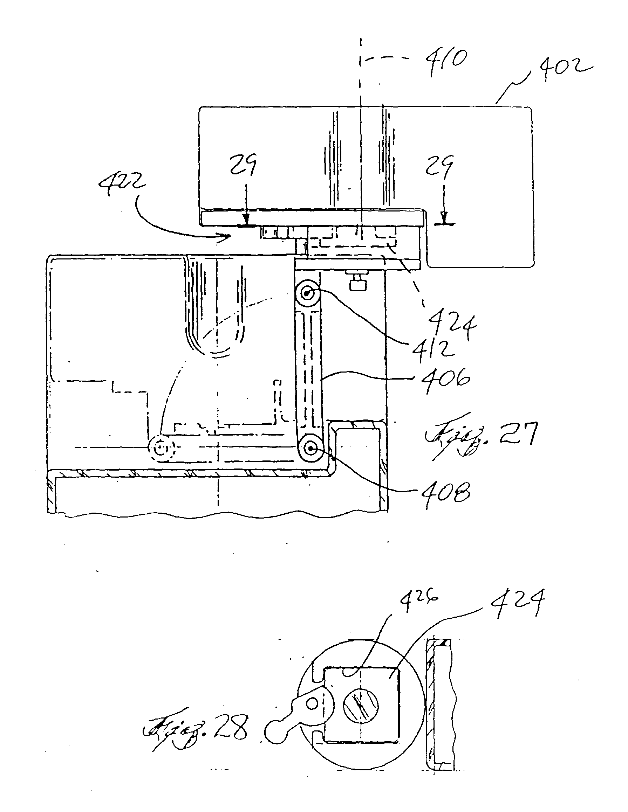

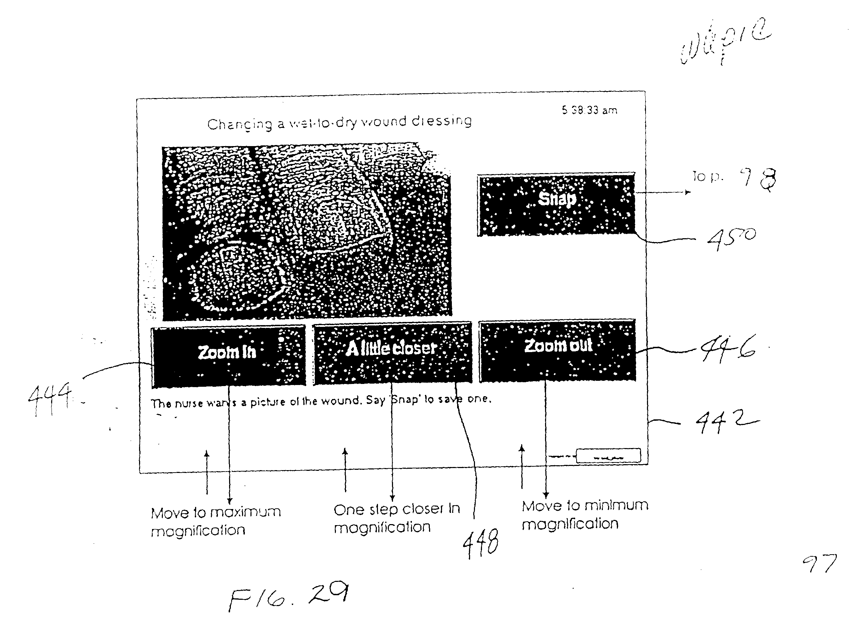

Still another embodiment of the patient monitoring and assistance device 10 is shown in Figs. 24-28. In this embodiment, the patient is visually monitored by means of a camera 400 that is contained in a module 402 nested into a recess 404 formed in the top of the device 10. The camera module 402, in turn, is detachably mounted to the end of an articulated arm assembly 406 mounted within the recess 404. The lower end of the articulated arm 406 is pivotable around a first substantially horizontal axis 408 extending substantially across the width of the device 10. The upper end of the arm 406 permits rotary movement of the camera module 402 around a substantially vertical axis 410 as well as around a second substantially horizontal axis 412. The three axis movement thus provided by the articulated arm 406 thus permits the camera module 402 to be pointed in virtually any direction.

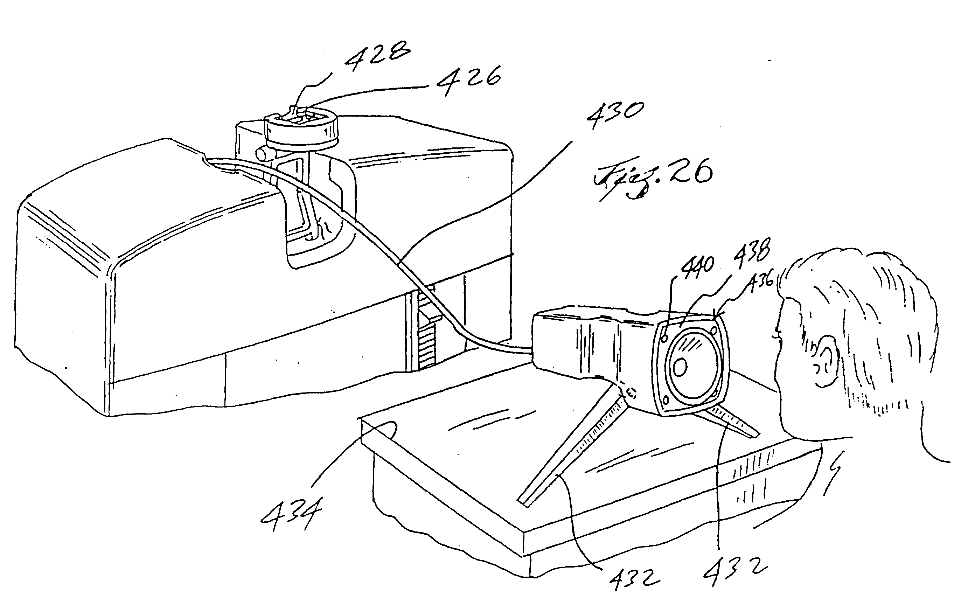

As best seen in Fig. 25, the recess 404 is of substantially square cross-section, and the camera module 402 is shaped and dimensioned so as substantially to fill the recess 404 when the module is nested therein. The top surface 414 of the camera module 402 is curved to match the upper surface 416 of the device 10. A pair of complementary finger grooves 418 formed in the sides of the camera module 402 and the recess 404 permit the module to be grasped and pulled upwardly from the nested or storage position to the use position shown in Fig. 24.

The upper end of the articulated arm 406 includes a base 420 on which the camera module 402 is mounted. The base 420 permits the camera module 402 to pivot around the vertical axis 410. The base 420 is also pivotally mounted to the upper end of the articulated arm 406 to permit pivoting movement around the upper horizontal axis 412.

The base 420 further includes a disconnect mechanism 422 best seen in Figs. 26-28. The disconnect mechanism 422 illustrated includes a square plate 424 affixed to the underside of the camera module 402 and a complementary-shaped recess 426 formed in the base 420. A pivotable key or cam 428 mounted on the base adjacent the recess 426 can be rotated in one direction to engage the plate 424 and thereby secure the camera module 402 to the base 420, or in the opposite direction to disengage the plate 424 and thereby allow the camera module 402 to be separated from the base 420. Preferably, the base 420 comprises an "off the shelf" photographic base of known construction and includes the disconnect mechanism 422 as a standard feature.

Referring further to Fig. 26, the disconnect mechanism 422 allows the camera module 402 to be disconnected from the articulated arm 406 and moved somewhat away from the main unit 10. A cable 430 extending from the rear of the camera module 402 connects the camera with the main unit 10. The length of the cable 430 is sufficient to allow the camera module 402 to be moved away from the main unit over a reasonable distance, e.g. 5 or 6 feet. The ability to disconnect the camera module 402 from the articulated arm 406 is useful in situations where it is difficult for the patient properly to position the camera due to the nature or location of the patients body to be used. For example, patients with surgical incisions on their backsides that require visual inspection with the camera might find it easier and more convenient to lie in a bed rather than stand in front of the unit 10. The removability of the camera module 402 allows the patient to position him or herself as is most convenient and thereafter position the camera module as needed to view the desired body area.

The camera module 402 is preferably provided with pair of legs 432 that can be used to support the camera module 402 on a table, chair or other supporting surface 434. Preferably, the legs 432 are independently adjustable so that the camera module 402 can be positioned as desired, even on an uneven surface. In the illustrated embodiment, the legs 432 are each pivotally joined to a side of the camera module 402. Each leg, therefore, can be rotated to whatever position is needed to place the camera in the desired position.

The camera module 402 is further provided with a light ring 436 that encircles the camera lens and that, when actuated, provides light for illuminating the subject matter being visually inspected. The light ring 436 includes a bezel or lens 438 that overlies a plurality of light bulbs 440 mounted on the camera module 402. Illumination of the light ring 436 can be directly controlled by the patient, or can be remotely controlled by personnel at the central facility 28 if it is believed that more light is necessary. Alternatively or additionally, actuation of the light ring 436 can be controlled automatically by the unit 10 if insufficient light is detected. Power for illuminating the light bulbs 440 is supplied through the cable 430. The light ring 436 is illuminated continuously throughout the time light is needed and an image is being acquired.

The video capability provided by the camera module 402 can be used in many ways. As previously noted, the patient monitoring and assistance device 10 is operable to store digitized pictures obtained via the camera 400 and, if desired to transmit the pictures to the central station or facility 28. Because of bandwidth limitations imposed by the communications link between the unit 10 and the central facility 28, the unit preferably provides for the storage and transmission of both high resolution pictures as well as low resolution pictures. The high resolution picture capability is used when it is desired to record pictures of high resolution and wherein transfer time to the central facility 28 is of reduced importance. The low resolution feature is used when it is desired to transmit pictures to the central facility 28 quickly and the level of detail in the pictures is of reduced importance. As a general method of operation, pictures obtained at the initiative of the patient, which will be used for evaluative purposes, are obtained and recorded at high resolution. Such pictures can be downloaded to the central facility 28 in a relatively leisurely manner and transmission speed is of lesser importance. Pictures obtained at the initiative of the central facility, which are more likely to be used for purposes of a general check on the patient's well being are obtained at lower resolution as speed of transfer, rather than extreme detail, is of primary concern. Such "live" video, as a rule, does not require high resolution.

One manner in which the video capability can be effectively used is in connection with patient compliance. To verify that the patient has indeed complied with a prescribed treatment regime, the patient can be instructed to take an appropriate picture each time a prescribed medication or procedure is taken or performed. The resultant pictures can be stored for later use if a question of patient compliance ever arises.

Another manner in which the video capability can be used is in connection with performing prescribed procedures such as changing the dressing on a wound. Many of such procedures call for the patient to look for abnormal conditions, such as excessive redness, swelling, discharge or odor. Because it is often difficult for inexperienced patients to accurately distinguish abnormal conditions from normal ones, the video capability can be used by the patient to obtain a picture of a questionable condition and download the picture to the central facility 28 for consideration by trained personnel.

In the illustrated embodiment, the video capability is actively utilized in connection with pre-arranged protocols for changing one or more types of wound dressings, for example, polymeric, hydrogel, central line and wet-to-dry wound dressings. In each case, the patient is instructed, after various prompts, to inspect the wound for changes in appearance, odor, color, amount of drainage, etc. If an abnormal condition is noted by the patient and communicated to the unit 10, the unit notifies the central facility. Personnel at the central facility 28 can then direct the unit 10 to instruct the patient to obtain an image of the suspect wound condition. Alternatively, in some treatment programs, the patient is routinely directed to photograph the wound using the camera module 402 at regularly scheduled intervals. Preferably, a screen 442 such as that shown in Fig. 29 appears. The screen 442 includes touch sensitive fields that can be actuated by the patient and used to control such camera functions as zoom. In the illustrated embodiment, separate fields 444, 446 are provided for zooming the camera in or out. An additional field 448 is provided for zooming the camera in incrementally, one step at a time. Still another field 450 is provided for actually "snapping" the picture. Preferably, voice recognition circuitry is included in the unit 10 which allows the patient to "snap" the picture aurally by saying a pre-arranged command, such as "snap." This eliminates the need to actually touch the screen 442 and is useful when the patient is so positioned that it is awkward or impossible to touch the screen with the fingers.

The video capability provided by the camera module 402 can also be used in other procedures as well. For example, the camera can be used to verify the proper placement of leads when an EKG is taken. Similarly, the video capability can be used to monitor proper functioning of intravenous (IV) delivery systems and IV sites. Such IV status checks can be performed as part of a prescribed care program or can be initiated by the patient in the event an abnormal condition is suspected.

Preferably, the unit 10 includes temporary memory or other storage for storing acquired pictures until such time as they are downloaded to the central facility 28. Preferably, some pictures, such as those initiated by the patient, are only stored until they are transmitted to the central facility. Other pictures, such as those used to verify patient compliance with prescribed procedures, can be stored for predetermined periods of time, e.g. two weeks.

Preferably, the video images are electronically compressed for transmission to the central facility. Known compression technology, such as "PK-Zip," can be used in this regard. Preferably, industry standard protocols are used in acquiring, storing and transmitting the digitized pictures in order to facilitate compatibility with other existing or future developed technologies.

As noted, the video capability is primarily used in connection with checking the condition of various types of wounds and for enabling the input of trained, experienced personnel when the patient is in doubt as to the normality of a condition. It will be appreciated that the visual capability can be used advantageously in a variety of manners not explicitly stated herein and that the examples of such use herein described are meant to be illustrative rather than limiting.

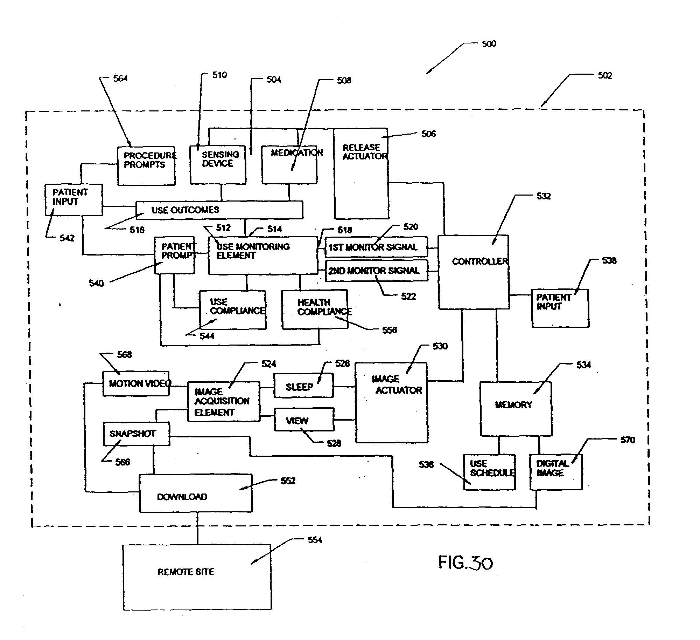

Fig. 30 diagrammatically shows a preferred embodiment of a highly interactive patient assistance device 500, which monitors patient compliance to preestablished physiologic health criteria as well as to preestablished self-care criteria. The device 500 includes a housing 502, which can take the form as shown in Figs. 1 and 2. One or more diagnostic or therapeutic items 504 usable by a patient are held by a first actuator 506. The actuator 506 retains the item(s) 504 on the housing 502. The actuator 506 operates in response to use command signals to selectively release the item(s) 504 for use by the patient.

Examples of such items 504 and the associated actuator 506 have already been described. For example, the item 504 can comprise medication 508, which is dispensed by an actuator of the kind shown in Fig. 3 in response to command signals generated as earlier described in detail and as shown in Figs. 5 to 9.

The item 504 can also comprise a sensing or testing device 510 that senses patient physiologic parameters, such a body temperature, pulse rate, blood glucose, etc. Examples of such testing device 510 have already been described in detail in this Specification.

As Fig. 30 shows, the device 500 includes, contained within the housing 502, a use monitoring element 512. The input 514 of the use monitoring element 512 receives use outcomes 516, which reflect use of one or more of the items 504 by the patient. The use monitoring element 512 includes an output 518, which generates monitoring signals based upon the use outcomes 516.

As Fig. 30 shows, the monitoring signals comprise a first monitoring signal 520, which is based upon a first use outcome. The monitoring signals also include a second monitoring signal 522, which is based upon a second use outcome different than the first use outcome.

As a general example, when the item 504 is medication 508, the second monitoring signal 522 is based upon a use outcome, which reflects that the patient has received and taken the medication as intended. In this example, the first monitoring signal 520 is based upon a different use outcome, which reflects that the patient has not taken the medication as intended, for example, because the patient has lost the medication or simply refuses to take it. Further examples of different use outcomes will appear later.

The device 500 further includes an image acquiring element 524 on the housing 502. The image acquiring element 524 can take various forms. In a preferred embodiment, the element 524 takes the form of the camera 400, the details of which have been previously described in conjunction with Figs. 24 to 28.

As Fig. 30 shows, the image acquiring element 524 is operable in a sleep mode 526, during which the element 524 acquires no image information. The image acquiring element is also operable in a view mode 528 to acquire a visual image of the patient. A second actuator 530 serves to switch operation of the image acquiring element 524 between the sleep mode 526 and the view mode 528.

The device 500 also includes a controller 532 on the housing 502. The controller 532 takes the form of a microprocessor controlled CPU as previously described in this Specification. The controller 532 is coupled to the use monitoring element 512 and the second actuator 530. The controller 532 commands switching of the image acquiring element 524 from the sleep mode 526 to the view mode 528 in response to the first monitoring signal 520 and not in response to the second monitoring signal 522.

The device 500 includes a transmitting element 552 to download the image acquired during the view mode to a remote site 554, such as the remote nursing station previously described (see Fig. 23). The image acquiring element 524 thereby forms a part of the overall interactive use and compliance monitoring functions of the device 500.

The controller 532 first governs release of a selected item 504 by the actuator 506. In a preferred embodiment, the device includes a memory 534 holding a prescribed use schedule 536 for the selected item 504. The controller 532 is coupled to the memory 534 and generates the use command signal to the first actuator 506 according to the prescribed use schedule. Details of schedule release of medication 508 or sensing devices 510 have already been described in this Specification.

Alternatively, or, preferably, in combination with scheduled release protocols, the device 500 includes a patient input 538. The input 538 receives prescribed information from the patient, requesting a release of the selected item 504. In this arrangement, the controller 532 is coupled to the patient input 538 and generates the use command signal to the first actuator 506 in response to input of such prescribed information from the patient.

The input 514 of the use monitoring element 512 receives use outcomes 516 from various sources. In one preferred embodiment, the use monitoring element 512 includes a prompting component 540. The prompting component 540 prompts the patient to provide use outcomes 516 by use of a patient input 542.

For example, if medication 508 is dispensed, the prompting component 540 asks the patient, either by audio or visual prompting (as already described): "Have you taken your medication?" The patient response, either yes or no or the absence of a response, comprises a type of use outcome 516.

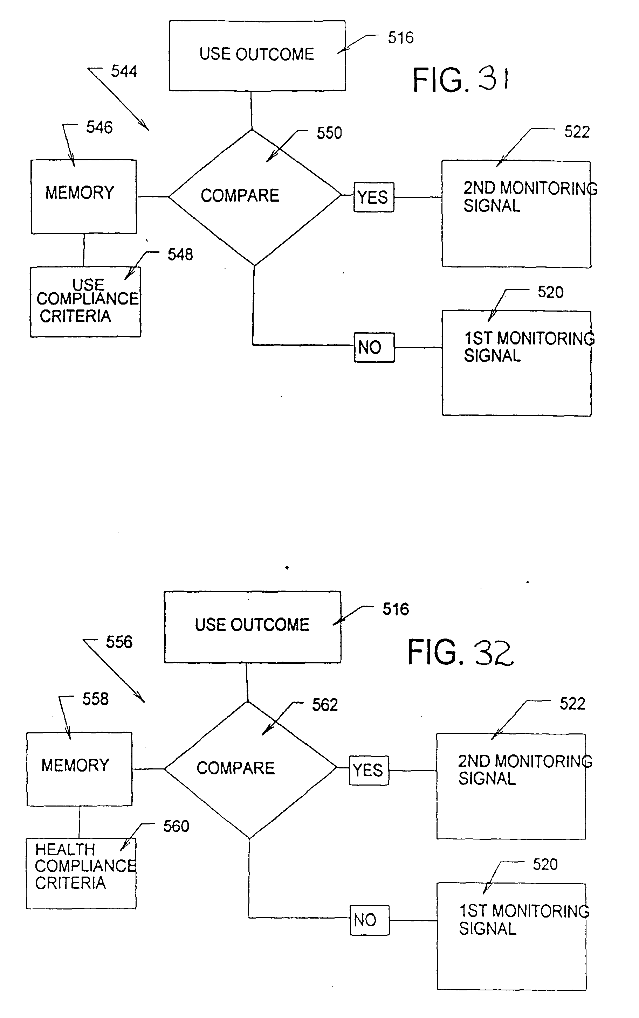

The use monitoring element output 518 includes a use compliance component 544 that includes memory 546 (see Fig. 31) to store prescribed use compliance criteria 548 for the item 504, which in the example is medication 508. The use compliance component 544 includes a comparator 550 to compare the use outcome 516 received by the use monitoring element input 514 to the prescribed use compliance criteria 548. The comparator 550 generates the first monitoring signal 520 if the use outcome 516 fails to comply with the prescribed use compliance criteria 548. The comparator 550 generates the second monitoring signal 522 if the use outcome 516 complies with the prescribed use compliance criteria 548.

In the context of the ongoing example, the "Yes" use outcome 516 received by the use monitoring element input 514 indicates compliance with preestablished use guidelines 548. The output 518 generates the second monitor signal 522, and the image acquisition element 524 remains in the sleep mode 526. The "No" or "No Response" use outcome 516 received by the use monitoring element input 514 indicates a lack of compliance with the preestablished use guidelines 548. The output 518 generates the first monitor signal 520. The controller 532 commands the actuator 530 to switch the image acquisition element 524 to the view mode 528. The image downloaded by the element 552 to the remote site 554 aids in the investigation why medication use compliance was not met.

For another example, if a sensing device 510 is the dispensed item 504, the prompting component 540 asks the patient, either by audio or visual prompting (as already described): "Do you have the sensing device?" The patient response, either yes or no or the absence of a response, comprises another type of use outcome 516. The "Yes" use outcome 516 indicates compliance with preestablished use guidelines 548, generating the second monitor signal 522 to keep the image acquisition element 524 in the sleep mode 526. The "No" or "No Response" use outcome 516 indicates a lack of compliance with the preestablished use guidelines 548, generating the first monitor signal 520 to switch the image acquisition element 524 to the view mode 528. The image downloaded by the element 552 to the remote site 554 again aids in the investigation why sensing device use compliance was not met.

Alteratively, the item 504 can itself generate use outcomes 516, either automatically or with patient action. For example, the sensing device 510 can include a switch requiring activation before use. Inactivation of the switch generates a first use outcome, equivalent to a "No" or "No Response" just described, and with the same result of switching the image acquiring element 524 to the view mode. Activation of the switch generates a second use outcome, equivalent to a "Yes" outcome just described, and with the same result of keeping the image acquiring element 524 in the sleep mode.

In the preferred embodiment (see Fig. 30), the use monitoring element output 518 also includes a health compliance monitoring component 556. As Fig. 32 shows, the health compliance monitoring component 556 includes memory 558 to store prescribed health compliance criteria 560 for the patient. A comparator 562 compares the use outcome 516 to the prescribed health compliance criteria 560. The comparator 562 generates the first monitoring signal 520 if the use outcome 516 fails to comply with the prescribed health compliance criteria 560. The comparator 562 generates the second monitoring signal 522 if the use outcome 516 complies with the prescribed health compliance criteria 560.

For example, if the dispensed item 504 is a sensing device 510 to sense temperature, the sensed temperatures comprise another type of use outcomes 516. In this arrangement, there is a first use outcome based upon a sensed normal temperature condition and a second use outcome based upon a different, sensed fever temperature condition.

The first use outcome 516 (normal temperature condition) indicates compliance with preestablished health compliance guidelines 560, generating the second monitor signal 522 to keep the image acquisition element 524 in the sleep mode 526. The second use outcome 516 (fever temperature condition) indicates a lack of compliance with the preestablished health criteria guidelines 560, generating the first monitor signal 520 to switch the image acquisition element 524 to the view mode 528. The image downloaded by the element 552 to the remote site 554 again aids in the investigation of the patient's physiologic condition.

Alternatively, or in combination with the above described protocol, the prompting component 540 can ask the patient: "Do you feel okay?" The "Yes", "No", or "No Response" answers constitute use outcomes 516. The "Yes" outcome 516 indicates compliance with preestablished health compliance guidelines 560, generating the second monitor signal 522 to keep the image acquisition element 524 in the sleep mode 526. The "No" or "No Response" outcome 516 indicates a lack of compliance with the preestablished health criteria guidelines 560, generating the first monitor signal 520 to switch the image acquisition element 524 to the view mode 528. The image downloaded by the element 552 to the remote site 554 again aids in the investigation of the patient physiologic condition.

As Fig. 30 shows, the device 500 also includes a component 564 that prompts the patient to follow a prescribed procedure, such as changing a wound dressing. This aspect of the device 500 has already been described in greater detail. As part of the procedure, the component 564 can ask the patient to provide one or more use outcomes 516 through the input 542. For example, the component 564 can ask: "Is the wound site sore to the touch?" The patient's responses -- "Yes" , "No", or "No Response" -- comprise a type of use outcome 516.

In the context of this example, the "No" outcome 516 indicates compliance with preestablished health compliance guidelines 560, generating the second monitor signal 522 to keep the image acquisition element 524 in the sleep mode 526. The "Yes" or "No Response" outcome 516 indicates a lack of compliance with the preestablished health criteria guidelines 560, generating the first monitor signal 520 to switch the image acquisition element 524 to the view mode 528. The image downloaded by the element 552 to the remote site 554 aids in the investigation of the patient physiologic condition.

As already described, and as shown in Fig. 30, the image acquisition element 524 is operable in a high resolution "snapshot" mode 566 and in a lower resolution, motion video mode 568. In a preferred embodiment, the first monitoring signals 520 differentiate patient circumstances to also command, via the controller 532, which image mode is entered at the time the controller 532 commands switching of the element 524 to the view mode.

For example, if the first monitoring signal 520 arises as a result of a "No Response" outcome 516, the monitoring signal 520 is coded so that the controller 532 commands the motion video mode 568 upon switching the image acquisition element 524 to the view mode 528. The resulting motion video image, when downloaded to the remote site 554, allows the remote site 554 to easily search for the patient in the vicinity of the device 500.

On the other hand, if the first monitoring signal 520 arises as a result of a "Yes" outcome 516 to the prompt "Is the wound site sore to the touch?", the monitoring signal 520 is coded so that the controller 532 commands the high resolution "snapshot" mode 568 upon switching the image acquisition element 524 to the view mode 528. The resulting "snapshot" image provides the resolution required to inspect the wound site for infection. The "snapshot" image can be downloaded immediately to the remote site 554. Alternatively, the "snapshot" image is temporarily stored in memory 534 as a digital image 570, for future downloading to the remote site 554 for analysis by trained medical personnel.

It is thus possible to supplement data relating to physiologic parameters of the patient taken by the sensing device 510 with digital images of the patient. For example, upon receiving a "Yes" outcome 516 to the prompt "Is the wound site sore to the touch?", the controller 532 can also command release of a sensing device 510 to sense the patient's temperature. An elevated body temperature sensed by the device 510 is indicative of an infection at the wound site. The existence of an infection at the wound site can be confirmed by a "snapshot" image acquired by the image acquiring element 524. Both forms of data, the temperature condition sensed by the device 510 and the digital image 520 can be stored in memory for later transmission and analysis by the remote site 554. Under the circumstances, the controller 532 can also command release of body temperature control medication 508, and further record in memory storage the dispensing of medication for this purpose.

It should be appreciated that the medication delivery systems described in this Specification are applicable for use out of association with a patient monitoring and assistance device. The systems can be used in virtually any environment where storage and delivery of selective medications are desired, such as in a hospital, nursing home, or pharmacy. It should also be appreciated that the medication delivery systems described can be actuated and controlled manually, without reliance upon the automated and highly interactive microprocessor controlled systems described in this Specification. Furthermore, each delivery mechanism and associated storage compartment can be used individually as a single unit, as well as in the multiple configurations shown in this Specification.

The features of the many aspects of the invention are set forth in the following claims.

## Claims

1. A patient assistance device comprising
a housing,
a monitoring element on the housing having an input to receive patient condition information regarding physiologic condition of a patient,
an image acquiring element on the housing operable to acquire an image of a body region of the patient, and
memory on the housing coupled to the monitoring element and the image acquiring element to store both the image and the patient condition information.

2. A device according to claim 1 and further including a transmitting element on the housing coupled either to the memory or the monitoring element or both to download patient condition information to a remote location.

3. A device according to claim 1 and further including a transmitting element on the housing coupled either to the image acquiring element or the memory or both to download the image to a remote location.

4. A device according to claim 1 wherein the image acquiring element is also selectively operable to acquire a motion video image of an exterior body region of the patient.

5. A patient assistance device comprising
a housing,
a prompting element on the housing to provide at least one prompt requiring a patient to provide input information in response to the prompt,
an input to receive the input information,
an image acquiring element on the housing operable in a sleep mode to acquire no images and in a view mode to acquire an image of a body region of
the patient, the image acquiring element including an actuator to switch operation of the image acquiring element between the sleep mode and the view mode, and
a controller that commands the actuator to switch the image acquiring element to the view mode when the input information meets prescribed criteria.

6. A patient assistance device comprising
a housing,
an image acquiring element on the housing operable in a sleep mode to acquire no images and in a view mode to acquire an image of a body region of the patient, the image acquiring element including an actuator to switch operation of the image acquiring element between the sleep mode and the view mode,
a component on the housing that instructs the patient to conduct a prescribed procedure and includes a prompting element to prompt the patient to provide input information in response to the prompt,
an input to receive the input information,
a controller that commands the actuator to switch the image acquiring element to the view mode when the input information meets prescribed criteria.

7. A device according to claim 5 or 6 and further including memory on the housing coupled to the image acquiring element to store the image.

8. A device according to claim 1, 5 or 7 wherein the image comprises a visual image of an exterior body region of the patient.

9. A device according to claim 1, 5 or 6 and further including a transmitting element to download the image to a remote location.

10. An apparatus for delivering health care services to a patient, comprising
a housing,
means within the housing for delivering
health care services to a patient, and
a video camera coupled to the housing and positionable by the patient for recording visual images of the patient.

11. An apparatus according to claim 10 wherein the video camera is contained within a module mounted on the housing.

12. An apparatus according to claim 11 wherein the module is coupled to the housing by means of an articulated arm.

13. An apparatus according to claim 12 wherein the module is detachable from the articulated arm.

14. An apparatus according to claim 13 wherein the articulated arm includes one end pivotally connected to the housing and an opposite end pivotally coupled to the module.

15. An apparatus according to claim 14 wherein the opposite end of the articulated arm is coupled to the module through a pivotable platform.

16. An apparatus according to claim 15 wherein the pivotable platform is detachable from the module.

17. An apparatus according to claim 13 or 16 wherein the module is electrically coupled to the housing through an elongate, flexible cable.

18. An apparatus according to claim 17 wherein the module nests into a recess formed in the housing.

19. An apparatus according to claim 18 wherein the recess is formed in the upper surface of the housing.

20. An apparatus according to claim 19 wherein the articulated arm is mounted to the housing within the recess.

21. An apparatus according to claim 20 wherein the module includes a pair of. finger grooves for facilitating removal of the module from the recess.

22. An apparatus according to claim 17 wherein the module is independently supportable on a surface when detached from the articulated arm.

23. An apparatus according to claim 22 wherein the module is supportable by means of legs extending from the module.

24. An apparatus according to claim 23 wherein the module includes two independently positionable legs extending from the module.

25. An apparatus according to any one of claims 11 to 24, wherein the module includes a light ring for illuminating the subject matter to be recorded by the camera.

26. An apparatus according to claim 25 wherein the camera includes a lens and wherein the light ring surrounds the lens.

27. An apparatus according to claim 26 wherein the light ring is continuously actuable so to continuously illuminate the subject matter to be recorded.

28. An apparatus according to claim 27 wherein the light ring is actuable in response to commands given by the patient.

29. An apparatus according to claim 27 wherein the light ring is actuable in response to commands received from a remote location.

30. An apparatus according to claim 27 wherein the light ring is actuable in response to commands generated within the apparatus.

31. An apparatus according to any one of claims 10 to 30 wherein the apparatus further includes means for storing a recorded image.

32. An apparatus according to claim 31 wherein the apparatus further includes means for transmitting the recorded image to an external monitoring facility.

33. An apparatus according to claim 31 wherein the apparatus further includes means for communicating instructions from the external monitoring facility back to the patient.

34. A patient assistance device comprising
a housing,
a diagnostic or therapeutic item usable by a patient,
a first actuator to retain the item on the housing and operating in response to a use command signal to release the item for use by the patient,
a use monitoring element having an input to receive use outcomes reflecting use of the item by the patient and an output to generate monitoring signals based upon the use outcomes, including a first monitoring signal based upon a first use outcome and a second monitoring signal based upon a second use outcome different than the first use outcome,
an image acquiring element on the housing operable in a sleep mode to acquire no visual image of the patient and in a view mode to acquire a visual image of the patient, the image acquiring element including a second actuator to switch operation of the image acquiring element between the sleep mode and the view mode, and
a controller on the housing coupled to the use monitoring element and the second actuator to command switching of the image acquiring element from the sleep mode to the view mode in response to the first monitoring signal and not in response to the second monitoring signal.

35. A device according to claim 34
and further including a memory holding a prescribed use schedule for the item, and
wherein the controller is coupled to the memory and generates the use command signal to the first actuator according to the prescribed use schedule.

36. A device according to claim 34
and further including a patient input for receiving prescribed information from the patient, and
wherein the controller is coupled to the patient input and generates the use command signal to the first actuator in response to input of prescribed information from the patient.

37. A device according to any one of claims 34 to 36,
wherein the item includes an output coupled to the use monitoring element input that generates use outcomes to the use monitoring element input.

38. A device according to any one of claims 34 to 36,
wherein the item includes medication for the patient.

39. A device according to any one of claims 34 to 38
wherein the use monitoring element includes a prompting component to prompt the patient to provide use outcomes to the use monitoring element input.

40. A device according to any one of claims 34 to 39
wherein the item includes a sensing device to sense conditions affecting the patient or physiologic parameters of the patient.

41. A device according to claim 40
wherein the sensing device includes an output coupled to the use monitoring element input to generate use outcomes indicating use of the sensing device to the use monitoring element input.

42. A device according to claim 40
wherein the sensing device includes an output coupled to the use monitoring element input that generates use outcomes indicating to the use monitoring element input conditions or physiologic parameters sensed by the sensing device.

43. A device according to any one of claims 34 to 42
wherein the use monitoring element output includes a use or health compliance component including memory to store prescribed use or health compliance criteria for the
item and a comparator to compare the use outcomes received by the use monitoring element input to the prescribed use or health compliance criteria and to generate the first monitoring signal if the use outcomes fail to comply with the prescribed use compliance
criteria and the second monitoring signal if the use outcomes comply with the prescribed use or health compliance criteria.

44. A device according to claim 43
wherein one of the prescribed use criteria requires a use outcome from the patient in response to the prompt.

45. A device according to claim 43 or 44,
wherein the item includes a sensing device having an input to sense conditions affecting the patient or physiologic parameters of the patient and an output coupled to the use monitoring element input to generate use outcomes based upon the conditions or physiologic parameters sensed by the sensing device, the use outcomes including the first use outcome based upon a first sensed condition and the second use outcome based upon a second sensed condition different than the first sensed condition, and
wherein one of the prescribed health compliance criteria requires a use outcome from the sensing device indicating a desired sensed condition affecting the patient or physiologic parameter for the patient.

46. A patient assistance device comprising
a housing,
a patient condition monitoring element on the housing having an input to receive patient condition information and an output to generate monitoring signals based upon patient condition,
an image acquiring element on the housing operable in a sleep mode to acquire no visual image of the patient and in a view mode to acquire a visual image of the patient, the image acquiring element including a second actuator to switch operation of the image acquiring element between the sleep mode and the view mode, and
a controller on the housing coupled to the patient condition monitoring element and the-second actuator to command switching of the image acquiring element from the sleep mode to the view mode in response to the monitoring signals.

47. A device according to claim 46 wherein the output of the patient condition monitoring element includes a first monitoring signal based upon a first patient condition and a second monitoring signal based upon a second patient condition different than the first patient condition, and
wherein the controller commands switching of the image acquiring element from the sleep mode to the view mode in response to the first monitoring signal and not in response to the second monitoring signal.

48. A device according to claim 46
wherein the patient condition monitoring element includes a prompting element to prompt the patient to provide patient condition information to the patient condition monitoring element input.

49. A device according to claim 46
wherein the patient condition monitoring element includes a sensing device having an input to sense physiologic parameters of the patient and an output to generate patient condition information to the patient condition monitoring element input.

50. A device according to claim 46
wherein the patient conditioning monitoring element includes a component that instructs the patient to conduct a prescribed procedure and includes a prompting element to prompt the patient to provide patient condition information to the patient condition monitoring element input during the prescribed procedure.

51. A device according to claim 46
wherein the controller includes an input to receive prescribed commands from the patient to command switching of the image acquiring element from the sleep mode to the view mode.

52. A patient assistance device comprising a housing and an image acquiring element on the housing operable in a first mode to acquire a motion video image of a patient having a first resolution and in a second mode to acquire a snapshot image of the patient having a second resolution greater than the f first resolution, the image acquiring element including an actuator to switch operation of the image acquiring element between the first mode and the second mode in response to a prescribed command.

53. A device according to claim 52 wherein the image acquisition element has a field of view and includes a light source to illuminate the field of view.

54. A device according to claim 52 wherein the image acquiring element has a field of view and includes a zoom element for changing the field of view.

55. A device according to claim 52 and further including memory on the housing to store the snapshot image.

56. A device according to claim 52 and further including a transmitting element on the housing to transmit the motion video image to a remote site for viewing.

57. A device according to claim 52 and further including a transmitting element on the housing to transmit the snapshot video image to a remote site for viewing.

58. A device according to claim 52 and further including a display on the housing to display to the patient images acquired by the image acquiring element.
